(19) Europäisches Patentamt
European Patent Office
Office européen des brevets

(11) **EP 4 238 951 B1**

(12) # EUROPEAN PATENT SPECIFICATION

(45) Date of publication and mention of the grant of the patent:
**03.12.2025 Bulletin 2025/49**

(21) Application number: **21884978.4**

(22) Date of filing: **19.10.2021**

(51) International Patent Classification (IPC):
***C07C 5/09*** $^{(2006.01)}$ ***C10G 70/02*** $^{(2006.01)}$
***C07C 11/167*** $^{(2006.01)}$ ***C07C 11/16*** $^{(2006.01)}$
***B01J 8/00*** $^{(2006.01)}$ ***B01J 8/02*** $^{(2006.01)}$
***B01J 19/00*** $^{(2006.01)}$ ***C10G 45/32*** $^{(2006.01)}$
***C10G 67/04*** $^{(2006.01)}$ ***C10G 21/00*** $^{(2006.01)}$

(52) Cooperative Patent Classification (CPC):
(C-Sets available)
**C07C 5/09; B01J 8/001; B01J 8/008; B01J 8/02; B01J 19/002; C10G 21/00; C10G 45/32; C10G 67/0427; C10G 70/02;** B01J 2208/0053; B01J 2208/00539; B01J 2208/00628 (Cont.)

(86) International application number:
**PCT/CN2021/124668**

(87) International publication number:
**WO 2022/089250 (05.05.2022 Gazette 2022/18)**

(54) **METHOD FOR SELECTIVE HYDROGENATION OF BUTADIENE EXTRACTION TAIL GAS AND SELECTIVE HYDROGENATION APPARATUS**

VERFAHREN ZUR SELEKTIVEN HYDRIERUNG VON BUTADIENEXTRAKTIONSENDGAS UND VORRICHTUNG ZUR SELEKTIVEN HYDRIERUNG

PROCÉDÉ D'HYDROGÉNATION SÉLECTIVE DE GAZ RÉSIDUAIRE D'EXTRACTION DE BUTADIÈNE ET DISPOSITIF D'HYDROGÉNATION SÉLECTIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2020 CN 202011158575**
**26.10.2020 CN 202011158581**
**26.10.2020 CN 202011156909**

(43) Date of publication of application:
**06.09.2023 Bulletin 2023/36**

(73) Proprietors:
- **China Petroleum & Chemical Corporation**
**Beijing 100728 (CN)**
- **BEIJING RESEARCH INSTITUTE OF CHEMICAL INDUSTRY,**
**CHINA PETROLEUM & CHEMICAL CORPORATION**
**Chaoyang District**
**Beijing 100013 (CN)**

(72) Inventors:
- **LI, Yan**
**Beijing 100013 (CN)**
- **TIAN, Jun**
**Beijing 100013 (CN)**
- **LI, Dongfeng**
**Beijing 100013 (CN)**
- **GUO, Liang**
**Beijing 100013 (CN)**
- **LI, Chunfang**
**Beijing 100013 (CN)**
- **YUE, Yi**
**Beijing 100013 (CN)**
- **DU, Zhou**
**Beijing 100013 (CN)**
- **SHU, Zhan**
**Beijing 100013 (CN)**
- **LUO, Shujuan**
**Beijing 100013 (CN)**
- **YE, Jieming**
**Beijing 100013 (CN)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

• **CUI, Ting**
**Beijing 100013 (CN)**

(74) Representative: **Hoffmann Eitle**
**Patent- und Rechtsanwälte PartmbB**
**Arabellastraße 30**
**81925 München (DE)**

(56) References cited:
**EP-A1- 1 217 060** **CN-A- 102 285 860**
**CN-A- 102 336 626** **CN-A- 103 121 905**

(52) Cooperative Patent Classification (CPC): (Cont.)

C-Sets
**C07C 5/09, C07C 11/16;**
**C07C 5/09, C07C 11/167**

## Description

### Technical Field

The present invention relates to the field of petrochemical industry, in particular to a method for selective hydrogenation of butadiene extraction tail gas and a selective hydrogenation apparatus thereof.

### Background Art

At present, butadiene extraction unit generally recovers 1,3-butadiene from a cracked C4 fraction of an ethylene unit through two-stage extractive distillation and ordinary distillation, and at the same time produces a by-product tail gas rich in alkynes and dienes. Due to the different technologies used in the butadiene extraction unit, the by-product alkynes-containing tail gas may be in two different states, i.e., liquid phase and gas phase, but the common feature thereof is relatively high concentration of vinyl acetylene (VA) and ethyl acetylene (EA) in the tail gas, in which the VA content is usually 20%, up to 40wt%. At present, in order to ensure safety, this tail gas rich in alkynes and dienes needs to be diluted with C4 raffinate, and then sold as liquefied gas, or directly discharged to the flare for combustion. If the tail gas is recycled and utilized, it will create good economic and social benefits.

At present, for the C4 resources rich in alkynes and dienes, the industry mainly converts them into high value-added products through hydrofining, in which one method used is selective hydrofining. Although hydrogenation activity of unsaturated hydrocarbons increases with the degree of unsaturation, and alkynes in the C4 components react with hydrogen gas preferentially over diolefins and monoolefins, the diolefins and monoolefins in the C4 components can also react violently with hydrogen gas at lower temperatures to form alkanes under the action of a catalyst. The selective hydrogenation reaction of C4 components is a three-phase reaction of gas, liquid and solid. However, due to small amount of hydrogen gas required for the reaction, hydrogen gas is limited to dissolve in C4 components, and then reacts with reactants such as alkynes and dienes in C4 components by mass transfer through the liquid membrane to the surface of catalyst. The inventors have found that hydrogenation reaction is limited by hydrogen gas, that is, on the surface of a catalyst, where hydrogen gas is insufficient, the alkynes cannot undergo the hydrogenation reaction, and thus hardly can be completely removed from the product, and polymerization reaction thereof is prone to occur to generate heavy components that may reduce the performance of the catalyst; and where hydrogen gas is excess, the diolefins and butenes produced by the hydrogenation reaction of alkynes may further undergo hydrogenation reactions to form alkenes and alkanes, resulting in a decrease in the selectivity of the hydrogenation reaction of alkynes.

CN102285859A discloses a method for selective hydrogenation of C4 streams, wherein by using palladium-silver two-component or palladium-silver multi-component catalysts with alumina as a carrier, the C4 streams with high butadiene content undergoes selective hydrogenation to obtain a product rich in 1-butene and having the content of butadiene and alkyne of less than 10ppm, which can be used as a raw material of MTBE plant. However, this patent does not involve a butadiene tail gas rich in vinyl acetylene (VA) and ethyl acetylene (EA), and hydrogen gas is directly allocated at the reactor inlet, the distribution of which is easily affected by the equipment and pipeline layout, so that it is difficult to ensure uniform distribution of hydrogen, which in turn limits selectivity of the catalyst.

CN103121905A discloses a recovering method for butadiene extraction tail gas, wherein by adopting a nickel-palladium-copper-silver multi-metal catalyst, alkynes undergo selectively hydrogenation to obtain a product rich in butadiene, which is sent to a butadiene unit for further recovery of butadiene. In this method, hydrogen gas is directly fed at the reactor inlet, the distribution of which is easily affected by equipment and pipeline layout, so that it is difficult to ensure uniform distribution of hydrogen, which in turn limits selectivity of the catalyst. In addition, since heavy components such as polymers produced by the selective hydrogenation reaction are not removed from the hydrogenated C4 stream that is used to dilute raw material, they may easily accumulate in the system, resulting in a reduced performance and life of the catalyst.

CN108863697A discloses a method for recovering butadiene by selective hydrogenation of alkynes, wherein by adopting a palladium-molybdenum selective hydrogenation catalyst, after hydrogenation of alkynes in the C4 stream, the vinyl acetylene content is lower than 1.0wt%, the butadiene selectivity is higher than 46%, and the product meets the feed requirements of the butadiene extraction unit. However, this patent uses a noble-metal-containing palladium catalyst and thus has a high cost, and it does not involve a process of selective hydrogenation unit for butadiene tail gas.

CN103787811A discloses a method for utilizing butadiene tail gas, wherein a Ni-based catalyst with a titanium oxide-alumina composite as carrier are adopted to hydrogenate all alkynes, dienes and monoenes in the tail gas, resulting in the product with the content of olefins of less than 5%, which can be used as raw material for cracking in ethylene plant, but this patent does not involve the field of selective hydrogenation.

CN109806885A discloses a Pdx/Cu single-atom catalyst for selective hydrogenation of C4 stream and its preparation method. After unsaturated olefins in C4 stream undergo hydrogenation, selectivity of total butenes is greatly improved, but the reaction temperature is relatively higher, resulting in more energy consumption; in addition, it does not disclose the process flow. Documents CN102336626A, CN102285860A and EP 1217060 disclose a method for the selective hydrogenation of C4-streams.

In recent years, there have been many researches on C4-component selective hydrogenation catalysts, and activity and selectivity of the catalysts have been greatly improved. However, as mentioned above, inaccurate control and uneven distribution of hydrogen gas severely limit the selectivity of the catalysts, making it difficult for the selective hydrogenation reaction to simultaneously meet the requirements of high conversion of alkynes and dienes and high yield of monoolefins. Compared with small pilot plants in the laboratory, in actual industrial plants, production scale has increased by a hundred times, precise control of hydrogen gas is more difficult and distribution of hydrogen gas is more uneven. According to characteristics of the industrial plants, it is more necessary to improve conditions of selective hydrogenation reaction in terms of process and control.

Therefore, there is still a need for a method for selective hydrogenation of butadiene extraction tail gas and a selective hydrogenation apparatus thereof, which can promote the even distribution of hydrogen gas during the selective hydrogenation reaction, improve the selectivity of the selective hydrogenation reaction, and prolong the service life of the catalyst.

## Contents of the Present Invention

The object of the present invention is to provide a method for selective hydrogenation of butadiene extraction tail gas and a selective hydrogenation apparatus thereof to solve the defects in the prior art. By improving the method for allocating and metering the hydrogen gas required for the selective hydrogenation reaction, the problem of uneven distribution of reactor temperature caused by uneven distribution of hydrogen, which in turn leads to a decrease in the selectivity of the hydrogenation reaction, can be solved. While ensuring the accurate metering of hydrogen, it promotes the even distribution in the selective hydrogenation reaction, improves the selectivity of the selective hydrogenation reaction, reduces the occurrence of side reactions, and prolongs the service life of the catalyst.

In order to achieve the above object, one aspect of the present invention provides a method for selective hydrogenation of butadiene extraction tail gas, characterized in that the selective hydrogenation method comprises:

(1) an alkyne-containing tail gas from a butadiene extraction unit is fed into a raw material tank, optionally impurities entrained in the alkyne-containing tail gas are removed before being fed into the raw material tank;

(2) a C4 raw material in the raw material tank is pressurized by a feed pump to a pressure required for reaction, then merged with a circulated C4 stream from a first-stage reactor outlet buffer tank and fed into a first-stage mixer, wherein it is mixed with hydrogen gas, and fed into the first-stage reactor to undergo a first-stage hydrogenation reaction, and a first-stage reaction stream obtained by the reaction is fed into the first-stage reactor outlet buffer tank;
the hydrogen gas required for the reaction in the first-stage reactor is fed through a first feeding mode or a second feeding mode:

the first feeding mode comprises: all the hydrogen gas required for the reaction is fed through the first-stage reactor outlet buffer tank, and then fed into the first-stage reactor through a first route at an outlet of the first-stage reactor outlet buffer tank;

the second feeding mode comprises: a part of the hydrogen gas required for the reaction is fed through the first-stage reactor outlet buffer tank, and then fed into the first-stage reactor through the first route at an outlet of the first-stage reactor outlet buffer tank; the other part of the hydrogen gas is fed through the first-stage mixer, and then fed into the first-stage reactor;

(3) there is no gas phase discharge from the first-stage reactor outlet buffer tank, the liquid-phase product is divided into at least two streams, wherein the first stream is returned to the first-stage reactor as the circulated C4 stream, and the second stream is used as a feed to a stabilization tower or subjected to further hydrotreatment prior to being fed to the stabilization tower;

(4) a C4 hydrogenation product is recovered after separation in the stabilization tower.

Another aspect of the present invention provides an apparatus for selective hydrogenation of butadiene extraction tail gas, which is used for carrying out the method for selective hydrogenation of butadiene extraction tail gas of the present invention, characterized in that the apparatus comprises: a raw material tank, a feed pump, a coalescer, a first-stage mixer, a first-stage reactor, a first-stage reactor outlet buffer tank, a circulated C4 cooler, a stabilization tower and a hydrogen gas feed pipeline;

the raw material tank, the feed pump, the coalescer, the first-stage mixer, the first-stage reactor and the first-stage reactor outlet buffer tank are connected in sequence;

an outlet pipeline of the first-stage reactor outlet buffer tank is divided into at least two routes, wherein the first route is connected with the circulated C4 cooler, the first-stage mixer and the first-stage reactor in sequence, and the second route is connected with the stabilization tower directly or indirectly;

the hydrogen gas feed pipeline is divided into at least a first pipeline and optionally a second pipeline, wherein the first pipeline is connected with the first-stage reactor outlet buffer tank, and the second pipeline is connected with the first-stage mixer;

preferably, the first-stage reactor is a fixed-bed reactor;

preferably, the first route is connected with the circulated C4 cooler via a circulation pump.

The technical solution of the present invention has at least the following beneficial effects:

By adopting the method and apparatus of the present invention, the butadiene extraction tail gas (alkyne-containing tail gas from butadiene extraction unit) can be fully and safely recycled; and by optimizing the hydrogen gas allocation and feeding mode of the selective hydrogenation process, and using the way of dissolving hydrogen gas to allocate and add hydrogen gas, the distribution of hydrogen gas in the selective hydrogenation reaction is effectively improved, the uneven reactor temperature distribution caused by the uneven distribution of hydrogen gas is overcome, and the selectivity of alkenes in the selective hydrogenation reaction of butadiene tail gas is improved;

in addition, by optimizing the dilution way of raw material and using C4 hydrogenation products with low impurity contents of 1,3-butadiene, butene-1 and heavy components to dilute the raw material, the present invention can solve the problem of high alkyne concentration in the raw material tank, solve the problem of high concentration of gas-phase alkyne tail gas in raw material pressurization, liquefaction and recovery, and also reduce the probability of rehydrogenation of 1,3-butadiene and butene-1 caused by back-mixing of the diluent C4 stream, thereby effectively reducing the product loss, improving the product yield, reducing the impact of heavy component impurities on the catalyst, and prolonging the service life of the catalyst;

in addition, the present invention can further remove the water-soluble impurities in the butadiene extraction tail gas raw material by providing a water-washing tower, so as to improve the adaptability of the raw material.

Other features and advantages of the present invention will be described in detail in the following description.

## Brief Description of the Drawings

Exemplary embodiments of the present invention will be described in more detail with reference to the accompanying drawings.

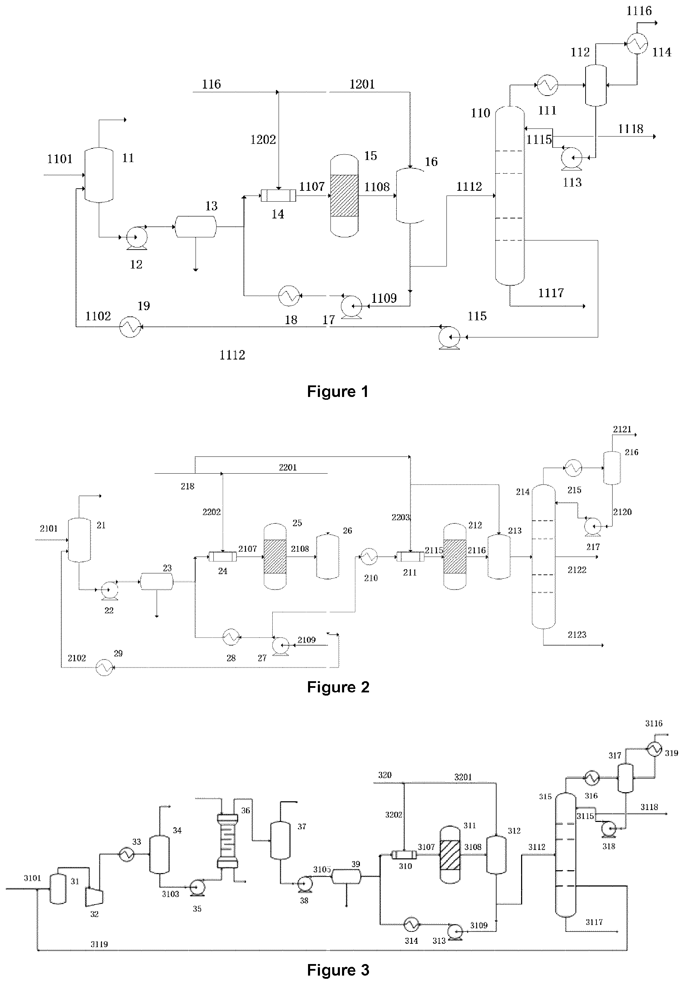

Figure 1 shows a schematic diagram of the method for selective hydrogenation of butadiene extraction tail gas according to an example of the present invention, wherein:

device signs are described as follows:
11, a raw material tank; 12, a feed pump; 13, a coalescer; 14, a first-stage mixer; 15, a first-stage reactor; 16, a first-stage reactor outlet buffer tank; 17, a circulation pump; 18, a circulated C4 cooler; 19, a diluent C4 cooler; 110, a stabilization tower; 111, a tower top condenser; 112, a reflux tank; 113, a reflux pump; 114, a tail gas condenser; 115, a diluent C4 pump; 116, a hydrogen gas feed pipeline;

stream signs are described as follows:
1101, an alkyne-containing tail gas (butadiene tail gas) from butadiene extraction unit; 1102, a diluent C4 stream; 1107, a first-stage reactor raw material; 1108, a first-stage reactor product; 1109, a circulated C4 stream; 1112, a stabilization tower feed; 1115, a stabilization tower reflux; 1116, a non-condensable gas; 1117, heavy components; 1118, a C4 hydrogenation product; 1201, a reaction pressure-compensating hydrogen gas; 1202, a reaction-supplement hydrogen gas.

Figure 2 shows a schematic diagram of the method for selective hydrogenation of butadiene extraction tail gas according to an example of the present invention, wherein:

device signs are described as follows:
21, a raw material tank; 22, a feed pump; 23, a coalescer; 24, a first-stage mixer; 25, a first-stage reactor; 26, a first-stage reactor outlet buffer tank; 27, a circulation pump; 28, a circulated C4 cooler; 29, a diluent C4 cooler; 210, a second-stage feed cooler; 211, a second-stage mixer; 212, a second-stage reactor; 213, a second-stage reactor outlet buffer tank; 214, a stabilization tower; 215, a tower top condenser; 216, a reflux tank; 217, a reflux pump; 218, a hydrogen gas feed pipeline;

stream signs are described as follows:
2101, an alkyne-containing tail gas (butadiene tail gas) from butadiene extraction unit; 2102, a diluent C4 stream; 2107, a first-stage reactor raw material; 2108, a first-stage reactor product; 2109, a circulated C4 stream; 2115, a second-stage reactor raw material; 2116, a second-stage reactor product; 2120, a stabilization tower reflux; 2121, a non-condensable gas; 2122, a C4 alkene product; 2123, heavy components; 2201, a reaction pressure-compensating hydrogen gas; 2202, a first-stage reaction mixed hydrogen gas; 2203, a second-stage reaction mixed hydrogen gas.

Figure 3 shows a schematic diagram of the method for selective hydrogenation of butadiene extraction tail gas according to an example of the present invention, wherein:

device signs are described as follows:
31, a blower suction tank; 32, a blower; 33, a liquefaction condenser; 34, a C4 collection tank; 35, a booster pump; 36, a water-washing tower; 37, a raw material tank; 38, a feed pump; 39, a coalescer; 310, a first stage mixer; 311, a first stage reactor; 312, a first-stage reactor outlet buffer tank; 313, a circulation pump; 314, a circulated cooler; 315, a stabilization tower; 316, a tower top condenser; 317, a reflux tank; 318, a reflux pump; 319, a tail gas condenser; 320, a hydrogen gas feed pipeline;

stream signs are described as follows:
3101, an alkyne-containing tail gas (butadiene tail gas) from butadiene extraction unit; 3103, a liquefied C4 raw material; 3105, a C4 feed; 3107, a first-stage reactor raw material; 3108, a first-stage reactor product; 3109, a circulated C4 stream; 3112, a stabilization tower feed; 3115, a stabilization tower reflux; 3116, a non-condensable gas; 3117, heavy components; 3118, a C4 hydrogenation product; 3119, a diluent C4 stream; 3201, a reaction pressure-compensating hydrogen gas; 3202, a reaction mixed hydrogen gas.

## Specific Models for Carrying Out the Present Invention

The present invention will be specifically described below in conjunction with specific examples. It is necessary to point out that the following examples are only used to further illustrate the present invention, and cannot be interpreted as limiting the protection scope of the present invention. Some non-essential improvements and adjustments made by a person skilled in the art according to the present invention still belong to the protection scope of the present invention. In addition, it is noted that various specific technical features described in the following specific embodiments may be combined in any suitable manner if there is no contradiction. In order to avoid unnecessary repetition, various possible combinations are not further described in the present invention.

An aspect of the present invention is to provide a method for selective hydrogenation of butadiene extraction tail gas, characterized in that, the selective hydrogenation method comprises:

(1) an alkyne-containing tail gas from a butadiene extraction unit is fed into a raw material tank, optionally impurities entrained in the alkyne-containing tail gas are removed before being fed into the raw material tank;

(2) a C4 raw material in the raw material tank is pressurized by a feed pump to a pressure required for reaction, then merged with a circulated C4 stream from a first-stage reactor outlet buffer tank and fed into a first-stage mixer, wherein it is mixed with hydrogen gas, and fed into the first-stage reactor to undergo a first-stage hydrogenation reaction, and a first-stage reaction stream obtained by the reaction is fed into the first-stage reactor outlet buffer tank;
the hydrogen gas required for the reaction in the first-stage reactor is fed through a first feeding mode or a second feeding mode:

the first feeding mode comprises: all the hydrogen gas required for the reaction is fed through the first-stage reactor outlet buffer tank, and then fed into the first-stage reactor through a first route at an outlet of the first-stage reactor outlet buffer tank;

the second feeding mode comprises: a part of the hydrogen gas required for the reaction is fed through the first-stage reactor outlet buffer tank, and then fed into the first-stage reactor through the first route at an outlet of the first-stage reactor outlet buffer tank; the other part of the hydrogen gas is fed through the first-stage mixer, and then fed into the first-stage reactor;

(3) there is no gas phase discharge from the first-stage reactor outlet buffer tank, the liquid-phase product is divided into at least two streams, wherein the first stream is returned to the first-stage reactor as the circulated C4 stream, and the second stream is used as a feed to a stabilization tower or subjected to further hydrotreatment prior to being fed into the stabilization tower;

(4) a C4 hydrogenation product is recovered after separation in the stabilization tower.

In the present invention, "alkyne-containing tail gas from butadiene extraction unit" refers to a tail gas rich in alkynes and dienes produced by the butadiene extraction unit when recovering 1,3-butadiene from a cracked C4 fraction of an ethylene unit. The alkyne-containing tail gas from butadiene extraction unit can be used interchangeably with a butadiene extraction tail gas. The concentration of vinyl acetylene (VA) and ethyl acetylene (EA) in the tail gas is relatively high, wherein the content of VA is usually 20%, up to 40wt%. Due to the different technologies adopted by the butadiene extraction unit, the generated alkyne-containing tail gas comprises two states, liquid phase and gas phase.

<u>Step (1)</u>

In an embodiment, the alkyne-containing tail gas from butadiene extraction unit may be directly fed into the raw material tank.

Alkynes such as vinyl acetylene in the alkyne-containing tail gas have the characteristics of self-decomposition and explosion, which will pose a safety risk. Therefore, it is generally necessary to dilute the tail gas to reduce its concentration and prevent explosion. According to the present invention, materials containing low contents of 1,3-butadiene, butene-1 and heavy components that tend to affect the activity and service life of the catalyst may be used as diluents.

In an embodiment, the alkyne-containing tail gas may be diluted with a side-draw diluent C4 stream from the stabilization column. Preferably, the mass flow ratio of the diluent C4 stream to the alkyne-containing tail gas is 1-30:1, for example 1-20:1, for example 1-10:1. In the present invention, the side-draw diluent C4 stream from the stabilization tower mainly comprises low contents of 1,3-butadiene, butene-1 and heavy components that tend to affect activity and service life of the catalyst.

In an embodiment, the alkyne-containing tail gas may also be diluted with a diluent C4 stream from the first-stage reactor outlet buffer tank. Preferably, the mass flow ratio of the diluent C4 stream to the alkyne-containing tail gas is 1-5:1, for example, 1-4:1.

The choice of the diluent C4 stream from the stabilization tower or the diluent C4 stream from the first-stage reactor outlet buffer tank mainly depends on the catalyst used in the selective hydrogenation reaction and the content of heavy components contained in the diluent C4 stream.

In the present invention, the alkyne-containing tail gas may also entrain impurities such as acetonitrile (ACN), N-methylpyrrolidone (NMP) and N,N-dimethylformamide (DMF). In order to reduce the impact of these impurities on subsequent processes, the alkyne-containing tail gas may be treated to remove the impurities entrained therein before being fed into the raw material tank. For example, a water-washing tower may be set up to remove water-soluble impurities in the alkyne-containing tail gas, so as to improve the adaptability of raw materials.

In an embodiment, the alkyne-containing tail gas may be fed into a water-washing tower to remove the impurities entrained in the alkyne-containing tail gas, and then fed into the raw material tank.

In an embodiment, when the alkyne-containing tail gas is a gas-phase alkyne-containing tail gas, the gas-phase alkyne-containing tail gas may be pressurized and liquefied into a liquid-phase alkyne-containing tail gas, and then fed into a water-washing tower. For example, the alkyne-containing tail gas is fed into a blower suction tank, pressurized by a blower, condensed and liquefied by a liquefaction condenser, and then fed into a C4 collection tank, followed by pressurization by a booster pump and being fed into a water-washing tower to remove the impurities entrained in the alkyne-containing tail gas.

According to the present invention, in the case that the gas-phase alkyne-containing tail gas undergoes a treatment of impurities removal, the alkyne-containing tail gas in the blower suction tank may be diluted.

In an embodiment, the gas-phase alkyne-containing tail gas in the blower suction tank may be diluted with a side-draw gas-phase C4 hydrogenation product from the stabilization tower. Preferably, the mass flow ratio of the gas-phase C4 hydrogenation product to the gas-phase alkyne-containing tail gas is 1-30:1, such as 1-20:1, such as 1-10:1.

In an embodiment, the gas-phase alkyne-containing tail gas in the blower suction tank may also be diluted with a diluent C4 stream from the first-stage reactor outlet buffer tank. Preferably, the mass flow ratio of the diluent C4 stream to the alkyne-containing tail gas is 1-5:1, for example, 1-4:1.

The choice of the diluent C4 stream from the stabilization tower or the diluent C4 stream from the first-stage reactor outlet buffer tank mainly depends on the catalyst used in the selective hydrogenation reaction and the content of heavy components contained in the diluent C4 stream. According to the present invention, the raw material tank has an operating pressure of 0.5-1.0 MPaG.

According to the present invention, preferably, the blower suction tank has an operating pressure of 0-20KPa.

According to the present invention, the condensed and liquefied gas in the liquefaction condenser has a temperature of 0-20°C.

According to the present invention, the liquefaction condenser has a pressure of 50-100Kpa.

According to the present invention, the water-washing tower has an operating pressure of 0.5-1.0 MPaG.

According to the present invention, the mass ratio of the washing water to the C4 raw material is 1-5:1 in the water-washing tower.

Step (2)

After the C4 raw material in the raw material tank is pressurized by the feed pump to the required pressure for the reaction, it merges with the circulated C4 stream from the first-stage reactor outlet buffer tank and enters the first-stage mixer. After being mixed with hydrogen gas in the first-stage mixer, it enters the first-stage reactor to perform the first-stage hydrogenation reaction, and the first-stage reaction stream obtained by the reaction enters the first-stage reactor outlet buffer tank. Preferably, the C4 raw material in the raw material tank is a diluted C4 raw material.

According to the present invention, the hydrogen gas required for the first-stage reactor reaction is allocated and fed through a first feeding mode or a second feeding mode.

In the first feeding mode, all the hydrogen gas required for the reaction is fed into the first-stage reactor through the first-stage reactor outlet buffer tank. In the second feeding mode, a part of the hydrogen gas required for the reaction is fed into the first-stage reactor through the first-stage reactor outlet buffer tank, and the other part of the hydrogen gas required for the reaction is fed into the first-stage reactor through the first-stage mixer.

According to the present invention, in the first feeding mode, the hydrogen gas required by the first-stage hydrogenation reactor controls the pressure of the reaction system through pressure compensation, and all the hydrogen gas enters the liquid-phase C4 stream through the way of dissolution, and then is fed with the circulated C4 stream from the first-stage reactor into the first-stage hydrogenation reactor. In the second feeding mode, a part of the hydrogen gas required for the reaction is fed through the first-stage reactor outlet buffer tank. This part of hydrogen gas controls the pressure of the reaction system through pressure compensation, and at the same time enters into the liquid-phase C4 stream through the way of dissolution, and then is fed with the circulated C4 stream from the first-stage reactor into the first-stage hydrogenation reactor.

In the present invention, by adopting the way of dissolving hydrogen gas to partially or completely replace the way of directly introducing hydrogen gas into an inlet of the reactor in the prior art, even distribution of hydrogen gas during the hydrogenation reaction is ensured, thereby improving the selectivity of the hydrogenation reaction.

In the second feeding mode, the mass ratio of the part of hydrogen gas required for the reaction to the hydrogen gas required for the first-stage hydrogenation reaction is not less than 0.3, preferably not less than 0.5, and the hydrogen gas required for the first-stage hydrogenation reaction is a sum of the part of hydrogen gas required for the reaction and the other part of hydrogen gas.

In the second feeding mode, the sum of the amount of the part of hydrogen gas required for the reaction and the amount of the other part of hydrogen gas is equal to the amount of the all hydrogen gas required for the hydrogenation reaction.

According to the present invention, the C4 raw material in the raw material tank is pressurized to 1.0-4.0 MPaG by the feed pump, and the mass flow ratio of the circulated C4 stream to the C4 raw material is 5-30:1. The inlet temperature of the first-stage reactor is 5-60°C, for example, 20-60°C; the liquid space velocity is 1-50$h^{-1}$; the pressure of the first-stage reactor is controlled by a pressure-compensating hydrogen gas of the first-stage reactor outlet buffer tank; and the reaction pressure is 1.0-4.0MPaG.

Preferably, the first-stage reactor is a fixed-bed reactor. The reactor is filled with a selective hydrogenation catalyst.

According to the present invention, a selective hydrogenation catalyst in the prior art, preferably the selective hydrogenation catalyst disclosed in CN102240547, may be used in the selective hydrogenation reaction. Based on the total weight of the catalyst, the palladium-containing catalyst preferably comprises the following components: 0.015 to 2.00wt% of palladium, 0.005 to 3.0wt% of a promoter metal, and a carrier as the balance, wherein the promoter metal is at least one selected from lead, silver, tin, magnesium and calcium, and the carrier is at least one selected from aluminum oxide, titanium oxide and magnesium oxide.

According to the present invention, the selective hydrogenation reaction may also use the selective hydrogenation catalysts disclosed in the prior art, such as CN102886262, CN10886397 and/or CN104707622, preferably the selective hydrogenation catalyst disclosed in CN104707622. Based on the total weight of the catalyst, the palladium-free catalyst preferably comprises the following components: 5-15 wt% of copper, 0.1-3 wt% of iridium, 0.1-3 wt% of phosphorus, 0.5-3.0 wt% of a promoter metal, and a carrier as the balance, wherein the promoter metal is at least one selected from nickel, zirconium, lead and tin, and the carrier is at least one selected from alumina, titania, silica, titania-alumina composite oxide, titania-silica composite oxide and alumina- silica composite oxide.

For the production of 1,3-butadiene, both the palladium-containing catalyst and the palladium-free catalyst may be used. Compared with the palladium-free catalyst, the palladium-containing catalyst has higher selectivity and higher olefin yield. However, since the palladium-containing catalyst contains noble metal, the investment and operating costs of the installation using the palladium-containing catalyst will be higher than those using the palladium-free catalyst. The use of catalysts that do not contain precious metals may effectively reduce investment and operating costs of the installation. Therefore, when choosing a catalyst, the relationship between cost and olefin yield needs to be weighed. For the production of butene-1, the palladium-containing catalyst is chosen due to its much higher selectivity and olefin yield.

Step (3)

There is no gas-phase discharge from the first-stage reactor outlet buffer tank, and a liquid-phase product is divided into at least two streams, wherein the first stream is returned to the first-stage reactor as the circulated C4 stream; the second stream is directly fed into the stabilization tower as a feed, or the second stream is further hydrotreated prior to being fed into the stabilization tower.

In the present invention, it is considered whether the second stream of the first-stage reactor outlet buffer tank needs to be further hydrotreated according to the demand of the product. For example, when a higher content of 1,3-butadiene is required in the product, the second stream may be used as a feed and directly fed into the stabilization column without further hydrogenation. When a higher content of butene-1 but a lower content of 1,3-butadiene is required in the product, the second stream needs to be further hydrotreated before being fed into the stabilization column.

Further hydrotreatment of the second stream before being fed into the stabilization tower comprises: the second stream is used as a feed to the second-stage reactor and fed to the second-stage reactor through the second-stage feed cooler and the second-stage mixer to carry out the second-stage hydrogenation reaction, and then a second-stage reaction stream obtained from the reaction in the second-stage reactor passes through a second-stage reactor outlet buffer tank and enters the stabilization tower.

Similar to the first-stage reactor, the hydrogen gas required for the second-stage reactor reaction is allocated and fed through a third feeding mode or a fourth feeding mode. In the third feeding mode, all the hydrogen gas required for the reaction is fed through the first-stage reactor outlet buffer tank, and then fed into the second-stage reactor through a second route at the outlet of the first-stage reactor outlet buffer tank. In the fourth feeding mode, a part of the hydrogen gas required for the reaction is fed through the first-stage reactor outlet buffer tank, and then fed into the second-stage reactor through the second route at the outlet of the first-stage reactor outlet buffer tank; and the other part of the hydrogen gas is fed through the second-stage mixer, and then fed into the second-stage reactor.

In the third feeding mode, the hydrogen gas required by the second-stage reactor controls the pressure of the first-stage reaction system through pressure compensation, and all the hydrogen gas enters the liquid-phase C4 stream through the way of dissolution, and is fed into the second-stage reactor with the feed to the second-stage reactor. In the fourth feeding mode, the part of the hydrogen gas required for the reaction is fed through the first-stage reactor outlet buffer tank. This part of hydrogen gas controls the pressure of the first-stage reaction system through pressure compensation, and at the same time enters the liquid-phase C4 stream by the way of dissolution, and is fed into the second-stage reactor with the feed to the second-stage reactor.

In the fourth feeding mode, the mass ratio of the part of the hydrogen gas required for the reaction to the hydrogen gas required for the second-stage hydrogenation reaction is not less than 0.3, preferably not less than 0.5, and the hydrogen gas required for the second-stage hydrogenation reaction is a sum of the part of the hydrogen gas required for the reaction and the other part of hydrogen.

In the fourth feeding mode, the sum of the amount of the part of hydrogen gas required for the reaction and the amount of the other part of hydrogen gas is equal to the amount of all the hydrogen gas required for the second-stage reaction.

In an embodiment, in order to produce butene-1, a two-stage hydrogenation process may be used for the alkyne-containing tail gas, wherein the alkynes and dienes in the alkyne-containing tail gas are selectively hydrogenated to produce monoolefins by theway of dissolving hydrogen gas, and then light and heavy components are removed by the stabilization tower to obtain a high-quality C4 product.

The first-stage reactor and the second-stage reactor each independently has an inlet temperature of 20-60°C; the first-stage reactor has a liquid space velocity of 10-50$h^{-1}$, and the second-stage reactor has a liquid space velocity of 1-10$h^{-1}$; the first-stage reactor and the second-stage reactor each independently has a pressure of 1.0-4.0MPaG, that is controlled by a pressure-compensating hydrogen gas of each reactor outlet buffer tank.

The first-stage reactor and the second-stage reactor are both fixed-bed reactors, and the reactors are filled with selective hydrogenation catalysts. As described above, the palladium-containing catalyst is chosen for the production of butene-1, due to its much higher selectivity and olefin yield.

The first-stage selective hydrogenation reaction and the second-stage selective hydrogenation reaction may use the selective hydrogenation catalysts in the prior art, preferably the selective hydrogenation catalyst disclosed in CN102240547. Based on the total weight of the catalyst, the palladium-containing catalyst preferably comprises the following components: 0.015-2.00wt% of palladium, 0.005-3.0wt% of a promoter metal, and a carrier as the balance, wherein the promoter metal is at least one selected from lead, silver, tin, magnesium and calcium, and the carrier is at least one selected from aluminum oxide, titanium oxide and magnesium oxide.

Step (4)

The C4 hydrogenation product is recovered after separation in the stabilization tower, and the C4 hydrogenation product comprises 1,3-butadiene and butene-1.

According to the present invention, when adopting an one-stage hydrogenation process to produce 1,3-butadiene, a condenser, preferably a two-stage condenser, is used at the top of the stabilization tower to recover the C4 components entrained in the non-condensable gas, a liquid phase recovery of the hydrogenation product with high contents of 1,3-butadiene and butene-1 is carried out by a reflux tank at the top of the tower, heavy components are removed from the tower kettle, and the C4 hydrogenation product with low contents of 1,3-butadiene, butene-1 and heavy components that tend to adversely affect catalyst activity and lifetime is obtained as a side-draw. The side-draw product may be used to dilute the C4 tail gas raw material. The phase state of the side-draw product of the stabilization tower is gas phase or liquid phase.

According to the present invention, when adopting a two-stage hydrogenation process to produce butene-1, the C4 product at the outlet of the second-stage reactor (the second-stage reaction stream obtained by the reaction in the second-stage reactor) enters the second-stage reactor outlet buffer tank, and the C4 stream from the bottom of the buffer tank enters the stabilization tower. A non-condensable gas is removed from the top of the stabilization tower, no liquid phase is obtained at the top of the tower, and total reflux is adopted; heavy components are removed from the bottom of the tower; and a high-quality C4 olefin product is obtained as a side-draw.

The stabilization tower has an operating pressure of 0.4-1.2MPaG, a theoretical plate number of 10-40, and a theoretical plate position for recovering a side-draw at 5-35.

In the present invention, after the liquid-phase butadiene tail gas is diluted and pressurized, a catalyst such as noble metal-free catalyst is used for the selective hydrogenation reaction, and light and heavy components are removed through the stabilization tower to obtain a C4 olefin product with low content of alkyne and enriched 1,3-butadiene, which may be returned to the butadiene extraction unit for further recovery of 1,3-butadiene and monoolefins.

In a specific embodiment, the present invention provides a method for selective hydrogenation of butadiene extraction tail gas, characterized in that, the selective hydrogenation method comprises:

(1) an alkyne-containing tail gas from a butadiene extraction unit is fed into a raw material tank;

(2) the raw material in the raw material tank is pressurized by a feed pump to the pressure required for the reaction, then merged with a circulated C4 stream from a first-stage reactor outlet buffer tank and fed into a first-stage mixer, wherein it is mixed with hydrogen gas and fed into a first-stage reactor to undergo a hydrogenation reaction, and a first-stage reaction stream obtained by the reaction is fed into the first-stage outlet buffer tank;
the hydrogen gas required for the first-stage reactor reaction is fed through a first feeding mode or a second feeding mode:

the first feeding mode comprises: all the hydrogen gas required for the reaction is fed through the first-stage reactor outlet buffer tank, and then fed into the first-stage reactor through a first route at an outlet of the first-stage reactor outlet buffer tank;

the second feeding mode comprises: a part of the hydrogen gas required for the reaction is fed through the first-stage reactor outlet buffer tank, and then fed into the first-stage reactor through the first route at an outlet of the first-stage reactor outlet buffer tank; and the other part of the hydrogen gas is fed through the first-stage mixer, and then fed into the first-stage reactor;

(3) there is no gas-phase discharge from the first-stage reactor outlet buffer tank, and a liquid phase product is divided into two streams, wherein the first stream is returned to the first-stage reactor as the circulated C4 stream, and the second stream is used as a feed to the stabilization tower;

(4) the first-stage reaction stream obtained from the reaction in the first-stage reactor is fed into the stabilization tower through the first-stage reactor outlet buffer tank, and separated through the stabilization tower to recover a C4 hydrogenation product.

In a preferred embodiment, in step (1), the alkyne-containing tail gas is diluted with a side-draw diluent C4 stream from the stabilization tower, preferably, the mass flow ratio of the diluent C4 stream and the alkyne-containing tail gas is 1 to 30: 1.

In a preferred embodiment, in step (2), in the second feeding mode, the mass ratio of the part of the hydrogen gas required for the reaction to the hydrogen gas required for the hydrogenation reaction is not less than 0.3, preferably not less than 0.5, and the hydrogen gas required for the hydrogenation reaction is a sum of the part of the hydrogen gas required for the reaction and the other part of the hydrogen gas.

In a preferred embodiment, in step (1), the raw material tank has an operating pressure of 0.5-1.0MPaG;

in step (2), the diluted C4 raw material is pressurized by the feed pump to 1.0-4.0MPaG, the mass flow ratio of the circulated C4 stream to the diluted C4 raw material is 5-30:1, the first-stage reactor has an inlet temperature of 5-60°C, and a liquid space velocity of 1-40h-1, the pressure of the first-stage reactor is controlled by a pressure-compensating hydrogen gas of the first-stage reactor outlet buffer tank, and the reaction pressure is 1.0-4.0MPaG;

in step (4), the stabilization tower has an operating pressure of 0.4-1.0MPaG, a theoretical plate number of 10-40, and a theoretical plate position for recovering a side-draw at 5-35.

In a specific embodiment, the present invention provides a method for selective hydrogenation of butadiene extraction tail gas, characterized in that, the selective hydrogenation method comprises:

(1) an alkyne-containing tail gas from a butadiene extraction unit is fed into a raw material tank, and the alkyne-containing tail gas in the raw material tank is diluted with a diluent C4 stream from a first-stage reactor outlet buffer tank;

(2) the diluted raw material in the raw material tank is pressurized by a feed pump to a pressure required for the reaction, then mixed with a circulated C4 stream from the first-stage reactor outlet buffer tank and fed into a first-stage mixer, wherein it is mixed with hydrogen gas, and then fed into the first-stage reactor to undergo a first-stage hydrogenation reaction, and the first-stage reaction stream obtained by the reaction is fed into the first-stage reactor outlet buffer tank;
the hydrogen gas required for the first-stage reactor reaction is fed through a first feeding mode or a second feeding mode:

the first feeding mode comprises: all the hydrogen gas required for the reaction is fed through the first-stage reactor outlet buffer tank, and then enters the first-stage reactor through a first route at an outlet of the first-stage reactor outlet buffer tank;

the second feeding mode comprises: a part of the hydrogen gas required for the reaction is fed through the first-stage reactor outlet buffer tank, and then enters the first-stage reactor through the first route at an outlet of the first-stage reactor outlet buffer tank; and the other part of the hydrogen gas is fed through the first-stage mixer, and then enters the first-stage reactor;

(3) there is no gas-phase discharge from the first-stage reactor outlet buffer tank, and a liquid phase product is divided into three streams, wherein the first stream is returned to the first-stage reactor as the circulated C4 stream, the second stream is used as a feed to the second-stage reactor and fed into a second-stage reactor through a second-stage feed cooler and a second-stage mixer to carry out a second-stage hydrogenation reaction; and the third stream is used as a diluent C4 stream and fed into the raw material tank;
the hydrogen gas required for reaction in the second-stage reactor is fed through a third feeding mode or a fourth feeding mode:

the third feeding mode comprises: all the hydrogen gas required for the reaction is fed through the first-stage reactor outlet buffer tank, and then enters the second-stage reactor through a second route at an outlet of the first-stage reactor outlet buffer tank;

the fourth feeding mode comprises: a part of the hydrogen gas required for the reaction is fed through the first-stage reactor outlet buffer tank, and then enters the second-stage reactor through a second route at an outlet of the first-stage reactor outlet buffer tank; and the other part of the hydrogen gas is fed through the second-stage mixer, and then enters the second-stage reactor;

(4) a second-stage reaction stream obtained from the reaction in the second-stage reactor is fed into the stabilization tower through the second-stage reactor outlet buffer tank, and separated through the stabilization tower to recover a C4 olefin product as a side-draw.

In a preferred embodiment, in step (2), in the second feeding mode, the mass ratio of the part of the hydrogen gas required for the reaction to the hydrogen gas required for the first-stage hydrogenation reaction is not less than 0.3, preferably not less than 0.5, and the hydrogen gas required for the hydrogenation reaction is a sum of the part of the hydrogen gas required for the reaction and the other part of the hydrogen gas.
In a preferred embodiment, in step (3), in the fourth feeding mode, the mass ratio of the part of the hydrogen gas required for the reaction to the hydrogen gas required for the second-stage hydrogenation reaction is not less than 0.3, preferably not less than 0.5, and the hydrogen gas required for the second-stage hydrogenation reaction is a sum of the part of the hydrogen gas required for the reaction and the other part of the hydrogen gas.
In a preferred embodiment, in step (1), the raw material tank has an operating pressure of 0.5-1.0 MPaG, and the mass flow ratio of the diluent C4 stream to the alkyne-containing tail gas is 1-5:1;

in step (2), the diluted raw material is pressurized to 1.0-4.0MPaG by the feed pump, and the mass flow ratio of the circulated C4 stream to the diluted C4 raw material is 5-30:1;

in step (4), the stabilization tower has an operating pressure of 0.6-1.2MPaG, a theoretical plate number of 10-40, and a theoretical plate position for recovering a side-draw at 5-35;

wherein, the first-stage reactor and the second-stage reactor each independently has an inlet temperature of 20-60°C, the first-stage reactor has a liquid space velocity of 10-50$h^{-1}$, and the second-stage reactor has a liquid space velocity of 1-10 $h^{-1}$; the first-stage reactor and the second-stage reactor each independently has a pressure of 1.0-4.0MPaG that is controlled by a pressure-compensating hydrogen gas of each reactor outlet buffer tank.

In a specific embodiment, the present invention provides a method for selective hydrogenation of butadiene extraction tail gas, characterized in that, the selective hydrogenation method comprises:

(1) a gas-phase alkyne-containing tail gas from a butadiene unit is fed into a blower suction tank, and the gas-phase alkyne-containing tail gas is pressurized by a blower, condensed and liquefied by a liquefaction condenser, and then fed into a C4 collection tank, followed by pressurization by a booster pump and being fed into a water-washing tower to remove impurities entrained in the alkyne-containing tail gas and then fed into a raw material tank;

(2) the C4 raw material in the raw material tank is pressurized by a feed pump to a pressure required for the reaction, then merged with a circulated C4 stream from a first-stage reactor outlet buffer tank and fed into a first-stage mixer, wherein it is mixed with hydrogen gas, and fed into a first-stage reactor to undergo a hydrogenation reaction, and the first-stage reaction stream obtained by the reaction is fed into the first-stage reactor outlet buffer tank;
the hydrogen gas required for reaction in the first-stage reactor is fed through a first feeding mode or a second feeding mode:

the first feeding mode comprises: all the hydrogen gas required for the reaction is fed through the first-stage reactor outlet buffer tank, and then enters the first-stage reactor through a first route at an outlet of the first-stage reactor outlet buffer tank;

the second feeding mode comprises: a part of the hydrogen gas required for the reaction is fed through the first-stage reactor outlet buffer tank, and then enters the first-stage reactor through the first route at an outlet of the first-stage reactor outlet buffer tank; and the other part of the hydrogen gas is fed through the first-stage mixer, and then enters the first-stage reactor;

(3) there is no gas-phase discharge from the first-stage reactor outlet buffer tank, and a liquid phase product is divided into two streams, wherein the first stream is returned to the first-stage reactor as the circulated C4 stream, and the second stream is used as a feed to the stabilization tower;

(4) the first-stage reaction stream obtained from the reaction in the first-stage reactor is fed into the stabilization tower through the first-stage reactor outlet buffer tank, and separated through the stabilization tower to recover a C4 hydrogenation product.

In a preferred embodiment, in step (1), the gas-phase alkyne-containing tail gas in the blower suction tank is diluted with a side-draw gas-phase C4 hydrogenation product from the stabilization tower; preferably, the mass flow ratio of the gas-phase C4 hydrogenation product to the gas-phase alkyne-containing tail gas is 1-30:1.

In a preferred embodiment, in step (2), in the second feeding mode, the mass ratio of the part of the hydrogen gas required for the reaction to the hydrogen gas required for the hydrogenation reaction is not less than 0.3, preferably not less than 0.5, and the hydrogen gas required for the hydrogenation reaction is a sum of the part of the hydrogen gas required for the reaction and the other part of the hydrogen gas.

In a preferred embodiment, in step (1), the blower suction tank has an operating pressure of 0-20KPa; the condensed and liquefied gas in the liquefaction condenser has a temperature of 0-20°C, and the liquefaction condenser has a pressure of 50-100KPa; the water-washing tower has an operating pressure of 0.5-1.0MPaG, the mass ratio of the washing water in the water-washing tower to the C4 raw material is 1-5:1; and the raw material tank has an operating pressure of 0.5-1.0MpaG;

in step (2), the C4 raw material is pressurized to 1.0-4.0MPaG by the feed pump;

the first-stage reactor has an inlet temperature of 5-60°C, a liquid space velocity of 10-50$h^{-1}$; the first-stage reactor has a reaction pressure of 1.0-4.0MPaG that is controlled by a pressure-compensating hydrogen gas of the first-stage reactor outlet buffer tank;

in step (4), the stabilization tower has an operating pressure of 0.4-1.0MPaG, a theoretical plate number of 10-40, and a theoretical plate position for recovering a side-draw at 5-35.

Another aspect of the present invention provides an apparatus for selective hydrogenation of butadiene extraction tail gas, which is used for carrying out the method for selective hydrogenation of butadiene extraction tail gas of the present invention, characterized in that the apparatus comprises: a raw material tank, a feed pump, a coalescer, a first-stage mixer, a first-stage reactor, a first-stage reactor outlet buffer tank, a circulated C4 cooler, a stabilization tower and a hydrogen gas feed pipeline;

the raw material tank, the feed pump, the coalescer, the first-stage mixer, the first-stage reactor and the first-stage reactor outlet buffer tank are connected in sequence;

an outlet pipeline of the first-stage reactor outlet buffer tank is divided into at least two routes, wherein the first route is connected with the circulated C4 cooler, the first-stage mixer and the first-stage reactor in sequence, and the second route is directly or indirectly connected with the stabilization tower;

the hydrogen gas feed pipeline is divided into at least a first pipeline and optionally a second pipeline, wherein the first pipeline is connected with the first-stage reactor outlet buffer tank, and the second pipeline is connected with the first-stage mixer;

preferably, the first-stage reactor is a fixed-bed reactor;

preferably, the first route is connected with the circulated C4 cooler via a circulation pump.

In the present invention, the raw material tank is provided with a butadiene extraction tail gas feed port and a diluent C4 port.

In an embodiment, the diluent C4 port of the raw material tank may be connected with an outlet of the stabilization tower, preferably an outlet of the stabilization tower is connected with the diluent C4 port through a diluent C4 pump and/or a diluent C4 cooler.

In an embodiment, the diluent C4 port of the raw material tank may be connected with an outlet pipeline of the first-stage reactor outlet buffer tank, preferably, a diluent C4 cooler is further provided between the diluent C4 port of the raw material tank and the outlet pipeline of the first-stage reactor outlet buffer tank.

In an embodiment, a top outlet of the stabilization tower is sequentially connected with a tower top condenser, a reflux tank and a reflux pump, and an outlet of the reflux pump is connected with an inlet of the stabilization tower.

In an embodiment, a top outlet of the stabilization tower is sequentially connected with a tower top condenser, a reflux tank and a reflux pump, an outlet of the reflux tank is connected with a tail gas condenser, an outlet of the tail gas condenser is connected with an inlet of the reflux tank, and another outlet of the reflux tank is connected with an inlet of the stabilization tower through the reflux pump.

According to the present invention, the apparatus for selective hydrogenation of butadiene extraction tail gas may further comprise a blower suction tank, a blower, a liquefaction condenser, a C4 collection tank, a booster pump and a water-washing tower, which are connected in sequence, and a C4 raw material outlet of the water-washing tower is connected with the raw material tank.

In an embodiment, an outlet of the stabilization tower is connected with a diluent C4 port of the blower suction tank.

In an embodiment, the water-washing tower is provided at top with a washing water inlet and a C4 raw material outlet, and provided at bottom with a liquefied C4 raw material inlet and a washing water outlet, and the booster pump is connected with the liquefied C4 raw material inlet.

According to the present invention, the apparatus for selective hydrogenation of butadiene extraction tail gas may further comprises a second-stage feed cooler, a second-stage mixer, a second-stage reactor and a second-stage reactor outlet buffer tank, which are connected in sequence, and the second route of the outlet pipeline of the first-stage reactor outlet buffer tank is connected with the second-stage feed cooler, and the second-stage reactor outlet buffer tank is connected with the stabilization tower; preferably, the second route is connected with the second-stage feed cooler through a circulation pump; preferably, the second-stage reactor is a fixed-bed reactor.

In an embodiment, the hydrogen gas feed pipeline is divided into at least a first pipeline and optionally a second pipeline and a third pipeline, wherein the first pipeline is connected with the first-stage reactor outlet buffer tank, the second pipeline is connected with the first-stage mixer, and the third pipeline is connected with the second-stage mixer; preferably, the hydrogen gas feed pipeline may further comprise a pressure-compensating pipeline that is connected with the second-stage reactor outlet buffer tank.

In an embodiment, an outlet pipeline of the first-stage reactor outlet buffer tank is divided into two routes, wherein the first route is connected with the circulated C4 cooler, the first-stage mixer and the first-stage reactor in sequence, and the second route is directly connected with the stabilization tower.

In an embodiment, an outlet pipeline of the first-stage reactor outlet buffer tank is divided into three routes, wherein the first route is connected with the circulated C4 cooler, the first-stage mixer and the first-stage reactor in sequence, the second route is connected with the second-stage feed cooler, and the third route is connected with the diluent C4 port of the raw material tank; and the hydrogen gas feed pipeline is divided into at least a first pipeline and optionally a second pipeline and a third pipeline, wherein the first pipeline is connected with the first-stage reactor outlet buffer tank, the second pipeline is connected with the first-stage mixer, and the third pipeline is connected with the second-stage mixer.

In a specific embodiment, the present invention provides an apparatus for selective hydrogenation of butadiene extraction tail gas, which is used for carrying out the method for selective hydrogenation of butadiene extraction tail gas of the present invention, characterized in that the apparatus comprises: a raw material tank, a feed pump, a coalescer, a first-stage mixer, a first-stage reactor, a first-stage reactor outlet buffer tank, a circulated C4 cooler, a stabilization tower and a hydrogen gas feed pipeline;

the raw material tank, the feed pump, the coalescer, the first-stage mixer, the first-stage reactor and the first-stage reactor outlet buffer tank are connected in sequence;

an outlet pipeline of the first-stage reactor outlet buffer tank is divided into two routes, wherein the first route is connected with the circulated C4 cooler, the first-stage mixer and the first-stage reactor in sequence; and the second route is connected with the stabilization tower;

the hydrogen gas feed pipeline is divided into at least a first pipeline and optionally a second pipeline, wherein the first pipeline is connected with the first-stage reactor outlet buffer tank, and the second pipeline is connected with the first-stage mixer.

In a preferred embodiment, an outlet of the stabilization tower is connected with the diluent C4 port of the raw material tank.

In a preferred embodiment, an outlet of the stabilization tower, the diluent C4 pump, the diluent C4 cooler and the diluent C4 port of the raw material tank are connected in sequence.

In a preferred embodiment, the first route of the outlet pipeline of the reactor outlet buffer tank is connected with the circulated C4 cooler via a circulation pump.

In a specific embodiment, the present invention provides an apparatus for selective hydrogenation of butadiene extraction tail gas, which is used for carrying out the method for selective hydrogenation of butadiene extraction tail gas of the present invention, characterized in that the apparatus comprises: a raw material tank, a feed pump, a coalescer, a first-stage mixer, a first-stage reactor, a first-stage reactor outlet buffer tank, a circulated C4 cooler, a second-stage feed cooler, a second-stage mixer, a second-stage reactor, a second-stage reactor outlet buffer tank, a stabilization tower and a hydrogen gas feed pipeline;

the raw material tank, the feed pump, the coalescer, the first-stage mixer, the first-stage reactor and the first-stage reactor outlet buffer tank are connected in sequence;

the second-stage feed cooler, the second-stage mixer, the second-stage reactor, the second-stage reactor outlet buffer tank and the stabilization tower are connected in sequence;

the raw material tank is provided with a butadiene extraction tail gas inlet and a diluent C4 port;

an outlet pipeline of the first-stage reactor outlet buffer tank is divided into three routes, wherein the first route is connected with the circulated C4 cooler, the first-stage mixer and the first-stage reactor in sequence, the second route is connected with the second-stage feed cooler, and the third route is connected with the diluent C4 port of the raw material tank;

the hydrogen gas feed pipeline is divided into at least a first pipeline and optionally a second pipeline and a third pipeline, wherein the first pipeline is connected with the first-stage reactor outlet buffer tank, the second pipeline is connected with the first-stage mixer, and the third pipeline is connected with the second-stage mixer.

In a preferred embodiment, the first route of the outlet pipeline of the first-stage reactor outlet buffer tank is connected with the circulated C4 cooler through a circulation pump; and the second route of the outlet pipeline of the first-stage reactor outlet buffer tank is connected with the second-stage feed cooler through a circulation pump.

In a preferred embodiment, a diluent C4 cooler is further provided between an outlet of the first-stage reactor outlet buffer tank and the diluent C4 port of the raw material tank.

In a preferred embodiment, the hydrogen gas feed pipeline further comprises a pressure-compensating pipeline that is connected with the second-stage reactor outlet buffer tank.

In a specific embodiment, the present invention provides an apparatus for selective hydrogenation of butadiene extraction tail gas, which is used for carrying out the method for selective hydrogenation of butadiene extraction tail gas of the present invention, characterized in that the apparatus comprises: a blower suction tank, a blower, a liquefaction condenser, a C4 collection tank, a booster pump, a water-washing tower, a raw material tank, a feed pump, a coalescer, a first-stage mixer, a first-stage reactor, a first-stage reactor outlet buffer tank, a circulated cooler, a stabilization tower and a hydrogen gas feed pipeline;

the blower suction tank, the blower, the liquefaction condenser, the C4 collection tank, the booster pump and the water-washing tower are connected in sequence;

an C4 raw material outlet of the water-washing tower, the raw material tank, the feed pump, the coalescer, the first-stage mixer, the first-stage reactor, and the first-stage reactor outlet buffer tank are connected in sequence;

an outlet pipeline of the first-stage reactor outlet buffer tank is divided into two routes, wherein the first route is connected with the circulated cooler, the first-stage mixer and the first-stage reactor in sequence; and the second route is connected with the stabilization tower;

the hydrogen gas feed pipeline is divided into at least a first pipeline and optionally a second pipeline, wherein the first pipeline is connected with the first-stage reactor outlet buffer tank, and the second pipeline is connected with the first-stage mixer.

In a preferred embodiment, an outlet of the stabilization tower is connected with a diluent C4 port of the blower suction tank.

In a preferred embodiment, the water-washing tower is provided at top with a washing water inlet and a C4 raw material outlet, and provided at bottom with a liquefied C4 raw material inlet and a washing water outlet, and the booster pump is connected with the liquefied C4 raw material inlet.

In a preferred embodiment, the first route of the outlet pipeline of the first-stage reactor outlet buffer tank is connected with the circulated cooler through a circulation pump.

## Example

The present invention is further illustrated by the following examples.

### Example 1

As shown in Figure 1, this example provided an apparatus for selective hydrogenation of butadiene extraction tail gas, and the apparatus comprised: a raw material tank **11,** a feed pump **12,** a coalescer **13,** a first-stage mixer **14,** a first-stage reactor **15,** a first-stage reactor outlet buffer tank **16,** a circulation pump **17,** a circulated C4 cooler **18,** a diluent C4 cooler **19,** a stabilization tower **110,** a tower top condenser **111,** a reflux tank **112,** a reflux pump **113,** a tail gas condenser **114,** a diluent C4 pump **115** and a hydrogen gas feed pipeline **116;**

wherein, the raw material tank **11** was provided with a butadiene extraction tail gas inlet and a diluent C4 port, and the bottom of the raw material tank **11,** the feed pump **12,** the coalescer **13,** the first-stage mixer **14,** the first-stage reactor **15** and the first-stage reactor outlet buffer tank **16** were connected in sequence;

an outlet pipeline of the first-stage reactor outlet buffer tank **16** was divided into two routes, wherein the first route was connected with the circulated C4 cooler **18,** the first-stage mixer **14** and the first-stage reactor **15** in sequence; the second route was connected with the stabilization tower **110;** wherein, the first route of the outlet pipeline of the first-stage reactor outlet buffer tank **16** was connected with the circulated C4 cooler **18** through the circulation pump **17;**

the hydrogen gas feed pipeline **116** was divided into at least a first pipeline and optionally a second pipeline, wherein the first pipeline was connected with a top inlet of the first-stage reactor outlet buffer tank **16,** and the second pipeline was connected with the first-stage mixer **14;**

wherein, an outlet of the stabilization tower **110,** the diluent C4 pump **115,** the diluent C4 cooler **19** and the diluent C4 port of the raw material tank **11** were connected in sequence; a top outlet of the stabilization tower **110** was connected with the tower top condenser **111** and the reflux tank **112** in sequence; an outlet of the reflux tank **112** was connected with the tail gas condenser **114,** and an outlet of the tail gas condenser **114** was connected with an inlet of the reflux tank 112; another outlet of the reflux tank **112** was connected with the reflux pump **113,** and an outlet of the reflux pump **113** was connected with an inlet of the stabilization tower **110;**

wherein, the hydrogenation reactor **15** was a fixed-bed reactor.

The apparatus for selective hydrogenation of butadiene extraction tail gas of this example was used to carry out a method for selective hydrogenation of butadiene extraction tail gas, and the selective hydrogenation method comprised:

(1) An alkyne-containing tail gas **1101** from a butadiene extraction unit (based on the total weight of the alkyne-containing tail gas, the main components of the alkyne-containing tail gas were: 58.69% butene, 10.35% butadiene, 17.65% ethyl acetylene and 4.00% vinyl acetylene, 2.05% C5 and higher, 0.02% water) was fed into the raw material tank **11,** and the alkyne-containing tail gas in the raw material tank was diluted with the cooled diluent C4 **1102** that was from a side-draw of the stabilization tower **110;** wherein, the alkyne-containing tail gas **1101** had a flow rate of 1825kg/h, and a pressure of 0.8MPaG; the diluent C4 **1102** had a flow rate of 5000kg/h, after dilution, in the raw material tank **11,** the liquid-phase vinyl acetylene content was 5.32%, and the vinyl acetylene content in the gas phase was 4.75%.

(2) The diluted raw material in the raw material tank **11** was pressurized to 2.7MPaG by the feed pump, then merged with the circulated C4 stream **1109** from the first-stage reactor outlet buffer tank **16** and fed into the first-stage mixer **14,** wherein it was mixed with hydrogen gas (i.e., to obtain hydrogenation reactor raw material **1107),** and fed into the first-stage reactor **15** for hydrogenation reaction, and the first-stage reaction stream (hydrogenation reactor product **1108)** obtained by the reaction was fed into the first-stage reactor outlet buffer tank **16;** the circulated C4 stream **1109** has a flow rate of 45000kg/h, the mixed C4 feed had a flow rate of 46825kg/h, and a temperature of 20°C.

The hydrogen gas required for the reaction in the first-stage reactor **15** was allocated and fed through a first feeding mode;

The first feeding mode comprised: all the hydrogen gas required for the reaction was fed through the first-stage reactor outlet buffer tank **16,** and then fed into the first-stage reactor **15** through a first route at the outlet of the first-stage reactor outlet buffer tank **16;** wherein, the pressure of the reaction system was controlled by the hydrogen gas required for the reaction in the first-stage reactor **15** through pressure-compensation and the pressure was 2.3MPaG; the hydrogen gas was fed into the liquid-phase C4 stream through a way of dissolution and then was fed with the circulated C4 stream **1109** of the reactor into the hydrogenation reactor, the circulated C4 stream **1109** had a flow rate of 45000kg/h, the hydrogen gas dissolved therein had a flow rate of 34.0kg/h, hydrogen gas was no longer fed into the first-stage mixer **14** provided at the inlet of the first-stage reactor **15,** and the reaction liquid phase had a total space velocity of 15 $h^{-1}$; the first-stage reactor **15** had an inlet temperature of 20°C;

(3) The first-stage reactor outlet buffer tank **16** had no gas-phase discharge, and the liquid phase product was divided into two streams, wherein the first stream was returned to the hydrogenation reactor as the circulated C4 stream **1109,** and the second stream was used as the stabilization tower feed **1112;** the stabilization tower feed **1112** had a flow rate of 6853 kg/h;

(4) The first-stage reaction stream (the first-stage reactor product **1108)** obtained from the reaction in the first-stage reactor **15** was fed into the stabilization tower **110** through the first-stage reactor outlet buffer tank **16,** and separated by the stabilization tower **110** to produce a C4 hydrogenation product **1118;** wherein, the stabilization tower **110** was used to remove non-condensable gas **1116** from the tower top, to remove heavy components **1117** from the tower kettle, to produce a liquid-phase C4 hydrogenation product **1118** rich in 1,3-butadiene and monoolefins from the tower top, and to recover as a side-draw the diluent C4 stream **1102** that is used for diluting raw materials and at a flow rate of 5000kg/h. The stabilization tower **110** had a theoretical plate number of 30, an operating pressure of 0.5MPaG, a tower top temperature of 56.7°C, a tower kettle temperature of 100.2°C, a reflux (stabilization tower reflux **1115)** flow rate of 5600kg/h, and a theoretical plate position for recovering a side-draw at 25.

The results of main streams were shown in Table 1.

The catalyst used in Examples 1 and 2 was: based on the total weight of the catalyst, the catalyst was composed of the following components: copper at a content of 7wt%, iridium at a content of 0.3wt%, phosphorus at a content of 2wt%, nickel at a content of 3wt%, and a carrier as the balance, in which the carrier was a titania-alumina composite oxide.

Table 1

| Stream No. | 1101 | 1102 | 1108 | 1118 | 1201 | 1202 |
|---|---|---|---|---|---|---|
| Phase state | Liquid | Liquid | Liquid | Liquid | Gas | Gas |
| Temperature [°C] | 40.0 | 20.1 | 35.3 | 15.0 | 15.8 | 15.8 |
| Pressure [MPa(g)] | 0.80 | 0.65 | 2.30 | 0.50 | 2.40 | 2.40 |
| Molar composition | % | % | % | % | % | % |
| Hydrogen gas | 0.00 | 0.00 | 0.00 | 0.17 | 95.00 | 95.00 |
| Methane | 0.00 | 0.00 | 0.46 | 0.60 | 5.00 | 5.00 |
| Propane | 0.00 | 0.00 | 0.00 | 0.00 | 0.00 | 0.00 |
| Propylene | 0.01 | 0.00 | 0.00 | 0.01 | 0.00 | 0.00 |
| Propyne | 0.00 | 0.00 | 0.00 | 0.00 | 0.00 | 0.00 |
| n-Butane | 2.57 | 4.14 | 3.91 | 3.40 | 0.00 | 0.00 |
| iso-Butane | 4.67 | 0.84 | 1.87 | 4.75 | 0.00 | 0.00 |
| Butene-1 | 14.20 | 11.32 | 14.57 | 23.10 | 0.00 | 0.00 |
| iso-Butylene | 22.05 | 9.32 | 12.72 | 22.57 | 0.00 | 0.00 |
| cis-2-butene | 18.79 | 44.13 | 37.31 | 19.70 | 0.00 | 0.00 |
| trans-2-butene | 3.65 | 11.48 | 10.47 | 8.06 | 0.00 | 0.00 |
| 1,3-Butadiene | 6.35 | 9.30 | 10.78 | 16.29 | 0.00 | 0.00 |
| 1,2-Butadiene | 4.00 | 5.06 | 3.93 | 0.91 | 0.00 | 0.00 |

(continued)

| Stream No. | 1101 | 1102 | 1108 | 1118 | 1201 | 1202 |
|---|---|---|---|---|---|---|
| Ethyl acetylene | 4.00 | 0.31 | 0.26 | 0.12 | 0.00 | 0.00 |
| Vinyl acetylene | 17.63 | 0.76 | 0.64 | 0.31 | 0.00 | 0.00 |
| n-Pentane | 1.80 | 3.21 | 2.82 | 0.00 | 0.00 | 0.00 |
| C5+ | 0.25 | 0.06 | 0.27 | 0.00 | 0.00 | 0.00 |
| Water | 0.02 | 0.00 | 0.00 | 0.00 | 0.00 | 0.00 |
| Molar flow rate [kmol/h] | 32.78 | 88.56 | 1186.79 | 31.02 | 12.51 | 0.00 |
| Mass flow rate [kg/h] | 1825.00 | 5000.00 | 66825 | 1724.50 | 34.00 | 0.00 |

It can be seen from Table 1 that, in the product, the content of 1,3-butadiene was about 10% higher than that in the raw material, the content of alkynes was less than 0.5%, and the total conversion rate of alkynes was greater than 97%, which could meet the raw material requirements of the butadiene extraction unit.

Example 2

As shown in Figure 1, compared with Example 1, this example had the following differences:
In step (2), the hydrogen gas required for the reaction in the first-stage reactor **15** was allocated and fed through a second feeding mode; the second feeding mode comprised: a part of the hydrogen gas required for the reaction was fed through the first-stage reactor outlet buffer tank **16,** and then fed into the first-stage reactor **15** through a first route of an outlet of the first-stage reactor outlet buffer tank **16;** the other part of hydrogen gas was fed through the first-stage mixer **14,** and then entered the first-stage reactor **15;** wherein most of the hydrogen gas was fed through the first-stage reactor outlet buffer tank **16,** and this part of the hydrogen gas controlled the pressure of the reaction system through pressure compensation, and at the same time entered the liquid phase C4 stream through the way of dissolution, and was fed with the circulated C4 stream **1109** of the reactor into the first-stage reactor, the circulated C4 stream **1109** had a flow rate of 32000kg /h, and the hydrogen gas dissolved therein had a flow rate of 20.0kg/h; a small part of the hydrogen gas was fed through the first-stage mixer **14** provided at an inlet of the first-stage reactor **15,** and had a flow rate of 17.0kg/h.

The results of main streams were shown in Table 2.

Table 2

| Stream No. | 1101 | 1102 | 1108 | 1118 | 1201 | 1202 |
|---|---|---|---|---|---|---|
| Phase state | Liquid | Liquid | Liquid | Liquid | Gas | Gas |
| Temperature [°C] | 40 | 20 | 41.7 | 15 | 15.8 | 15.8 |
| Pressure [MPa(g)] | 0.8 | 0.65 | 2.3 | 0.5 | 2.4 | 2.4 |
| Molar composition | % | % | % | % | % | % |
| Hydrogen gas | 0 | 0 | 0 | 0.18 | 95 | 95 |
| Methane | 0 | 0 | 0.50 | 0.55 | 5 | 5 |
| Propane | 0 | 0 | 0.00 | 0.00 | 0 | 0 |
| Propylene | 0.01 | 0 | 0.00 | 0.01 | 0 | 0 |
| Propyne | 0 | 0 | 0.00 | 0.00 | 0 | 0 |
| n-Butane | 2.57 | 4.01 | 3.76 | 3.22 | 0 | 0 |
| iso-Butane | 4.67 | 0.86 | 1.88 | 4.75 | 0 | 0 |
| Butene-1 | 14.2 | 12.06 | 15.36 | 25.20 | 0 | 0 |
| iso-Butylene | 22.05 | 9.52 | 12.84 | 22.55 | 0 | 0 |
| cis-2-Butene | 18.79 | 44.78 | 37.78 | 19.61 | 0 | 0 |
| trans-2-Butene | 3.65 | 12.55 | 11.40 | 8.65 | 0 | 0 |
| 1,3-Butadiene | 6.35 | 8.76 | 10.07 | 14.12 | 0 | 0 |

(continued)

| Stream No. | 1101 | 1102 | 1108 | 1118 | 1201 | 1202 |
|---|---|---|---|---|---|---|
| 1,2-Butadiene | 4 | 4.06 | 3.16 | 0.72 | 0 | 0 |
| Ethyl acetylene | 4 | 0.41 | 0.34 | 0.13 | 0 | 0 |
| Vinyl acetylene | 17.63 | 0.96 | 0.80 | 0.33 | 0 | 0 |
| n-Pentane | 1.8 | 1.93 | 1.88 | 0.00 | 0 | 0 |
| C5+ | 0.25 | 0.10 | 0.22 | 0 | 0 | 0 |
| Water | 0.02 | 0 | 0 | 0 | 0 | 0 |
| Molar flow rate [kmol/h] | 32.78 | 88.87 | 695.1 | 30.75 | 7.36 | 6.26 |
| Mass flow rate [kg/h] | 1825 | 5000 | 38842 | 1710 | 20 | 17 |

It can be seen from Table 2 that, in the product, the content of 1,3-butadiene was about 7.8% higher than that in the raw material, the content of alkynes was less than 0.5%, and the total conversion rate of alkynes was greater than 97%, which could meet the raw material requirements of the butadiene extraction unit. Compared with Example 1, in this example, because the hydrogen gas was not fed into the reactor completely through the way of dissolution, even distribution of hydrogen gas was slightly worse than that of Example 1, which in turn caused a part of 1,3-butadiene to be consumed due to the hydrogenation reaction, resulting in a decrease in the increased value of the content of 1,3-butadiene in the product compared with the raw material.

Example 3

As shown in Figure 2, this example provided an apparatus for selective hydrogenation of butadiene extraction tail gas, and the apparatus comprised: a raw material tank **21,** a feed pump **22,** a coalescer **23,** a first-stage mixer **24,** a first-stage reactor **25,** a first-stage reactor outlet buffer tank **26,** a circulated C4 cooler **28,** a circulation pump **27,** a diluent C4 cooler **29,** a second-stage feed cooler **210,** a second-stage mixer **211,** a second-stage reactor **212,** a second-stage reactor outlet buffer tank **213,** a stabilization tower **214,** a tower top condenser **215,** a reflux tank **216,** a reflux pump **217,** a hydrogen gas feed pipeline **218;**

wherein, the bottom of the raw material tank **21,** the feed pump **22,** the coalescer **23,** the first-stage mixer **24,** the first-stage reactor **25** and the first-stage reactor outlet buffer tank **26** were connected in sequence;

the second-stage feed cooler **210,** the second-stage mixer **211,** the second-stage reactor **212,** the second-stage reactor outlet buffer tank **213** and the stabilization tower **214** were connected in sequence;

the raw material tank **21** was provided with a butadiene extraction tail gas inlet and a diluent C4 port;

an outlet pipeline of the first-stage reactor outlet buffer tank **26** was divided into three routes, wherein the first route was connected with the circulated C4 cooler **28,** the first-stage mixer **27** and the first-stage reactor **25** in sequence; the second route was connected with the second-stage feed cooler **210;** the third route was connected with the diluent C4 **2102** port of the raw material tank **21;**

the hydrogen gas feed pipeline **218** was divided into at least a first pipeline and optionally a second pipeline and a third pipeline, wherein the first pipeline was connected with the first-stage reactor outlet buffer tank **26,** the second pipeline was connected with the first-stage mixer **24,** and the third pipeline was connected with the second-stage mixer **211,** wherein, the hydrogen gas feed pipeline also comprised a pressure-compensating pipeline connected with the second-stage reactor outlet buffer tank **213.**

Wherein, the first route of the outlet pipeline of the first-stage reactor outlet buffer tank **26** was connected with the circulated C4 cooler **28** through the circulation pump **27;**
the second route of the outlet pipeline of the first-stage reactor outlet buffer tank **26** was connected with the second-stage feed cooler **210** through the circulation pump **27.**
A diluent C4 cooler **29** was also provided between an outlet of the first-stage reactor outlet buffer tank **26** and the diluent C4 port of the raw material tank **21.**
Wherein, a top outlet of the stabilization tower **214** was connected with the top condenser **215,** the reflux tank **216** and the reflux pump **217** in sequence, and an outlet of the reflux pump **217** was connected with an inlet of the stabilization tower **214.**

Wherein, the first-stage reactor **25** and the second-stage reactor **212** were both fixed-bed reactors.

The apparatus for selective hydrogenation of butadiene extraction tail gas of this example was used to carry out a method for selective hydrogenation of butadiene extraction tail gas, and the selective hydrogenation method comprised the following steps:

(1) The alkyne-containing tail gas **2101** from butadiene extraction unit (based on the total weight of the alkyne-containing tail gas, main components of the alkyne-containing tail gas were: 58.69% butene, 10.35% butadiene, 17.65% ethyl acetylene and 4.00% vinyl acetylene, 2.05% C5 and higher, 0.02% water) was fed into the raw material tank **21,** and the alkyne-containing tail gas **2101** in the raw material tank **21** was diluted with the diluent C4 stream **2102** from the first-stage reactor outlet buffer tank **26;** wherein, the alkyne-containing tail gas **2101** had a flow rate of 1825kg/h, the raw material tank **21** had an operating pressure of 0.5MPaG, the diluent C4 stream **2102** had a flow rate of 3000kg/h; after dilution, in the raw material tank, the content of liquid phase vinyl acetylene was 6.85%, and the content of vinyl acetylene in the gas phase was 3.18%.

(2) The diluted raw material in the raw material tank **21** was pressurized by the feed pump **22** to 2.7MPaG, then mixed with the circulated C4 stream **2109** from the first-stage reactor outlet buffer tank **26,** and then fed into the first-stage mixer **24,** wherein it was mixed with hydrogen gas (i.e., to obtain the first-stage reactor raw material **2107),** and fed into the first-stage reactor **25** to carry out the first-stage hydrogenation reaction, and the first-stage reaction stream (the first-stage reactor product **2108)** obtained in the reaction was fed into the first-stage reactor outlet buffer tank **26;** wherein, the circulated C4 stream **2109** had a flow rate of 85000kg/ h, the mixed C4 feed had a flow rate of 89759kg/h, and the temperature was 35°C.
The hydrogen gas required for the reaction in the first-stage reactor **25** was allocated and fed through a first feeding mode; the first feeding mode comprised: all the hydrogen gas required for the reaction was fed through the first-stage reactor outlet buffer tank **26,** and then fed into the first-stage reactor **25** through the first route at the outlet of the first-stage reactor outlet buffer tank **26;** wherein, the hydrogen gas required for the reaction in the first-stage reactor **25** controlled the pressure of the reaction system through pressure compensation and the pressure was 2.2MPaG, and the hydrogen gas entered the liquid phase C4 stream by the way of dissolution, and was fed with the circulated C4 stream **2109** of the first-stage reactor **25** into the first-stage reactor **25,** the circulated C4 stream **2109** had a flow rate of 85000kg/h, the hydrogen gas dissolved therein had a flow rate of 48.2kg/h, and the hydrogen gas was no longer fed into the first-stage mixer **24.**

(3) The first-stage reactor outlet buffer tank **26** had no gas-phase discharge, and the liquid-phase product was divided into three streams, wherein the first stream was used as the circulated C4 stream **2109** and returned to the first-stage reactor **25,** the second stream was used as a second-stage reactor feed and fed into the second-stage reactor **212** through the second-stage feed cooler **210** and the second-stage mixer **211** to carry out the second-stage hydrogenation reaction, and the third stream was used as the diluent C4 stream **2102** and fed into the raw material tank **21;** wherein, the second stream used as the second-stage reactor feed (the second-stage reactor raw material **2115)** had a flow rate of 1807kg/h, and a temperature of 35°C. The hydrogen gas required for the reaction in the second-stage reactor **212** was allocated and fed through a third feeding mode; the third feeding mode comprised: all the hydrogen gas required for the reaction was fed through the first-stage reactor outlet buffer tank **26,** and then fed into the second-stage reactor **212** through a second route at the outlet of the first-stage reactor outlet buffer tank **26,** and this part of the hydrogen gas fed by the way of dissolution had a flow rate of 1.8kg/h; the second-stage mixer provided at the inlet of the second-stage reactor was no longer provided with hydrogen gas.

(4) The second-stage reaction stream (the second-stage reactor product **116)** obtained by the reaction in the second-stage reactor **212** was fed into the stabilization tower **214** through the second-stage reactor outlet buffer tank **213,** and separated by the stabilization tower **214** to recover a C4 olefin product **2122** as a side-draw; wherein, the second-stage reaction stream obtained by the reaction in the second-stage reactor was fed into the second-stage reactor outlet buffer tank, the pressure was controlled at 2.2MPaG, the C4 stream at the bottom of the second-stage reactor outlet buffer tank was fed into the stabilization tower to remove non-condensable gas **2121** from the tower top of the stabilization tower, to remove heavy components **2123** from the tower kettle, and to obtain a high-quality C4 olefin product **2122** as a side-draw, and the C4 olefin product **2122** had a flow rate of 1710kg/h. The stabilization tower **214** had a theoretical plate number of 20, a tower top temperature of 71.5°C, a tower kettle temperature of 126°C, and a reflux (the stabilization tower reflux **2120)** flow rate of 2500kg/h. The stabilization tower **214** had an operating pressure of 0.85 MPaG.

Wherein, the first-stage reactor **25** and the second-stage reactor **212** each independently had an inlet temperature of 40°C, the first-stage reactor had a liquid space velocity of $20h^{-1}$, and the second-stage reactor had a liquid space velocity of $3h^{-1}$; the first-stage reactor **25** and the second-stage reactor **212** each independently had a pressure of 2.20 MPaG that was controlled by a pressure-compensating hydrogen gas of each reactor outlet buffer tank.
The results of main streams were shown in Table 3.
The catalyst used in Examples 3 and 4 was: based on the total weight of the catalyst, the catalyst was composed of the following components: Pd at a content of 0.3 wt%, metallic silver at a content of 0.3 wt%, and a carrier as the balance, in which the carrier was alumina.
The calculation method of olefin yield in Examples 3 and 4 was carried out according to following Formula 1:

$$\textbf{olefin yield} = \frac{\textbf{content of butenes at outlet of second} - \textbf{stage reactor (mol\%)}}{\textbf{sum of contents of butenes} + \textbf{butadienes} + \textbf{alkynes in raw material (mol\%)}} \times \textbf{100\%}$$

Formula 1

in Formula 1:
butenes include butene-1, cis-2-butene, trans-2-butene and isobutene;
butadienes include 1,3-butadiene and 1,2-butadiene;
alkynes include ethyl acetylene and vinyl acetylene;
content of butenes at outlet of second-stage reactor (mol%): sum of the normalized mol% contents of butene-1, cis-2-butene, trans-2-butene and isobutene after deducting the contents of hydrogen gas and methane from outlet composition of the second-stage reactor.

Table 3

| Stream No. | 2101 | 2108 | 2116 | 2122 | 2201 |
|---|---|---|---|---|---|
| Gas phase fraction | 0.00 | 0.00 | 0.00 | 0.00 | 1.00 |
| Temperature [°C] | 40.0 | 46.9 | 50.4 | 74.0 | 15.8 |
| Pressure [MPa(g)] | 0.80 | 2.20 | 2.20 | 0.88 | 2.40 |
| Molar composition | % | % | % | % | % |
| Hydrogen gas | 0.00 | 0.26 | 0.37 | 0.00 | 95.00 |
| Methane | 0.00 | 1.64 | 1.69 | 0.05 | 5.00 |
| Propane | 0.00 | 0.00 | 0.00 | 0.00 | 0.00 |
| Propylene | 0.01 | 0.01 | 0.01 | 0.00 | 0.00 |
| propyne | 0.00 | 0.00 | 0.00 | 0.00 | 0.00 |
| n-Butane | 2.57 | 2.60 | 2.92 | 3.06 | 0.00 |
| iso-Butane | 4.67 | 4.52 | 4.52 | 4.62 | 0.00 |
| Betene-1 | 14.20 | 32.69 | 26.20 | 27.19 | 0.00 |
| iso-butene | 22.05 | 21.52 | 21.48 | 22.25 | 0.00 |
| cis-2-Butene | 18.79 | 19.89 | 22.59 | 23.65 | 0.00 |
| trans-2-Butene | 3.65 | 14.31 | 18.08 | 18.93 | 0.00 |
| 1,3-Butadiene | 6.35 | 0.10 | <30ppm | <30ppm | 0.00 |
| 1,2-Butadiene | 4.00 | 0.07 | | | 0.00 |
| Ethyl acetylene | 4.00 | 0.05 | <5ppm | <5ppm | 0.00 |
| Vinyl acetylene | 17.63 | 0.19 | <5ppm | <5ppm | 0.00 |
| n-Pentane | 1.80 | 1.81 | 1.80 | 0.24 | 0.00 |
| C5+ | 0.25 | 0.32 | 0.32 | 0.00 | 0.00 |
| Water | 0.02 | 0.00 | 0.00 | 0.00 | 0.00 |
| Molar flow rate [kmol/h] | 32.78 | 1605.46 | 32.36 | 30.39 | 17.74 |

(continued)

| Stream No. | 2101 | 2108 | 2116 | 2122 | 2201 |
|---|---|---|---|---|---|
| Mass flow rate [kg/h] | 1825.00 | 89758.80 | 1807.00 | 1710.00 | 48.20 |

It can be seen from Table 3 that, in the product, the content of alkynes was less than 5ppm, and the content of dienes was less than 30ppm. Based on the outlet composition of the second-stage reactor, the olefin yield of the selective hydrogenation reaction reached 99.5%.

Example 4

As shown in Figure 2, compared with Example 3, this example had the following differences:

In step (2), the hydrogen gas required for the reaction in the first-stage reactor **25** was allocated and fed through a second feeding mode; the second feeding mode comprised: a part of the hydrogen gas required for the reaction was fed through the first-stage reactor outlet buffer tank **26,** and then fed into the first-stage reactor **25** through the first route of the outlet of the first-stage reactor outlet buffer tank **26;** the other part of the hydrogen gas was fed through the first-stage mixer **24,** and then fed into the first-stage reactor **25;** wherein most of the hydrogen gas was fed through the first-stage reactor outlet buffer tank **26,** and this part of hydrogen gas controlled the pressure of the reaction system through pressure compensation, simultaneously entered the liquid-phase C4 stream by the way of dissolution, and was fed with the circulated C4 stream **2109** of the first-stage reactor **25** into the reactor, the circulated C4 stream **2109** had a flow rate of 52000kg/h, and the hydrogen gas dissolved therein had a flow rate of 29.0kg/h; a small part of the hydrogen gas is fed through the first-stage mixer **24** provided at the inlet of the first-stage reactor **25,** and had a flow rate of 23.0kg/h.

In step (3), the hydrogen gas required for the reaction in the second-stage reactor **212** was allocated and fed through a fourth feeding mode; the fourth feeding mode comprised: a part of the hydrogen gas required for the reaction was fed through the first-stage reactor outlet buffer tank **26,** and then fed into the second-stage reactor **212** through the second route of the outlet of the first-stage reactor outlet buffer tank **26,** and the hydrogen gas fed by the way of dissolution had a flow rate of 1.8kg/h; the other part of the hydrogen gas was fed through the second-stage mixer **211,** and then fed into the second-stage reactor **212;** wherein, the hydrogen gas fed through the second-stage mixer **211** at the inlet of the second-stage reactor **212** had a flow rate of 1.5 kg/h.

The results of main streams were shown in Table 4.

Table 4

| Stream No. | 2101 | 2108 | 2116 | 2122 | 2201 | 2202 | 2203 |
|---|---|---|---|---|---|---|---|
| Gas phase fraction | 0.00 | 0.00 | 0.00 | 0.00 | 1.00 | 1.00 | 1.00 |
| Temperature [°C] | 40.0 | 54.4 | 71.6 | 74.3 | 15.8 | 15.8 | 15.8 |
| Pressure [MPa(g)] | 0.80 | 2.20 | 2.20 | 0.88 | 2.40 | 2.40 | 2.40 |
| Molar composition | % | % | % | % | % | % | % |
| Hydrogen gas | 0.00 | 0.42 | 0.25 | 0.00 | 95.00 | 95.00 | 95.00 |
| Methane | 0.00 | 1.74 | 1.88 | 0.06 | 5.00 | 5.00 | 5.00 |
| Propane | 0.00 | 0.00 | 0.00 | 0.00 | 0.00 | 0.00 | 0.00 |
| Propylene | 0.01 | 0.01 | 0.01 | 0.01 | 0.00 | 0.00 | 0.00 |
| Propyne | 0.00 | 0.00 | 0.00 | 0.00 | 0.00 | 0.00 | 0.00 |
| n-Butane | 2.57 | 5.72 | 7.94 | 8.31 | 0.00 | 0.00 | 0.00 |
| iso-Butane | 4.67 | 5.16 | 5.16 | 5.27 | 0.00 | 0.00 | 0.00 |
| Betene-1 | 14.20 | 29.48 | 21.78 | 22.57 | 0.00 | 0.00 | 0.00 |
| iso-butene | 22.05 | 20.85 | 20.85 | 21.57 | 0.00 | 0.00 | 0.00 |
| cis-2-Butene | 18.79 | 19.86 | 22.34 | 23.42 | 0.00 | 0.00 | 0.00 |

(continued)

| Stream No. | 2101 | 2108 | 2116 | 2122 | 2201 | 2202 | 2203 |
|---|---|---|---|---|---|---|---|
| trans-2-Butene | 3.65 | 14.23 | 17.67 | 18.50 | 0.00 | 0.00 | 0.00 |
| 1,3-Butadiene | 6.35 | 0.08 | <30ppm | <30ppm | 0.00 | 0.00 | 0.00 |
| 1,2-Butadiene | 4.00 | 0.11 | | | 0.00 | 0.00 | 0.00 |
| Ethyl acetylene | 4.00 | 0.01 | <5ppm | <5ppm | 0.00 | 0.00 | 0.00 |
| Vinyl acetylene | 17.63 | 0.19 | <5ppm | <5ppm | 0.00 | 0.00 | 0.00 |
| n-Pentane | 1.80 | 1.80 | 1.80 | 0.28 | 0.00 | 0.00 | 0.00 |
| C5+ | 0.25 | 0.31 | 0.31 | 0.00 | 0.00 | 0.00 | 0.00 |
| Water | 0.02 | 0.00 | 0.00 | 0.00 | 0.00 | 0.00 | 0.00 |
| Molar flow rate [kmol/h] | 32.78 | 1016.49 | 32.27 | 30.32 | 10.67 | 8.46 | 0.55 |
| Mass flow rate [kg/h] | 1825.00 | 56775.06 | 1805.56 | 1710.00 | 29.00 | 23.00 | 1.50 |

It can be seen from Table 4 that, in the product, the content of alkynes and dienes was lower than 30ppm, and the olefin yield reached 93.0% based on the outlet composition of the second-stage reactor. In this example, because the hydrogen gas was not fed into the reactor completely through the way of dissolution, the even distribution of hydrogen gas was slightly worse than that of Example 3, which in turn caused some monoolefins to continuously undergo hydrogenation reaction with hydrogen gas to form alkanes, resulting in a slight decrease in the selectivity of olefins.

Example 5

As shown in Figure 3, the present example provided an apparatus for selective hydrogenation of butadiene extraction tail gas, and the apparatus comprised: a blower suction tank 31, a blower **32,** a liquefaction condenser **33,** a C4 collection tank **34,** a booster pump **35,** a water-washing tower **36,** a raw material tank **37,** a feed pump **38,** a coalescer **39,** a first-stage mixer **310,** a first-stage reactor **311,** a first-stage reactor outlet buffer tank **312,** a circulation pump **313,** a circulated cooler **314,** a stabilization tower **315,** a hydrogen gas feed pipeline **320,** a tower top condenser **316,** a reflux tank **317,** a reflux pump **318** and a tail gas condenser **319;**

wherein, the blower suction tank **31,** the blower **32,** the liquefaction condenser **33,** the C4 collection tank **34,** the booster pump **35** and the water-washing tower **36** were connected in sequence; the blower suction tank **31** was provided with a butadiene extraction tail gas inlet and a diluent C4 port;

a C4 raw material outlet of the water-washing tower **36,** the raw material tank **37,** the feed pump **38,** the coalescer **39,** the first-stage mixer **310,** the first-stage reactor **311** and the first-stage reactor outlet buffer tank **312** were connected in sequence; wherein, the water-washing tower **36** was provided at top with a washing water inlet and a C4 raw material outlet, and provided at bottom with a liquefied C4 raw material inlet and a washing water outlet, and the booster pump **35** was connected with the liquefied C4 raw material inlet;

the outlet pipeline of the first-stage reactor outlet buffer tank **312** was divided into two routes, wherein the first route was connected with the circulated cooler **314,** the mixer **310** and the hydrogenation reactor **11** in sequence; the second route was connected with the stabilization tower **315;** wherein, the first route of the outlet pipeline of the first-stage reactor outlet buffer tank **312** was connected with the circulated cooler **314** through the circulation pump **313;**

the hydrogen gas feed pipeline **320** was divided into at least a first pipeline and optionally a second pipeline, wherein the first pipeline was connected with the first-stage reactor outlet buffer tank **312,** and the second pipeline was connected with the first-stage mixer **310;**

wherein, an outlet of the stabilization tower **315** was connected with the diluent C4 port of the blower suction tank **31.**

Wherein, the top outlet of the stabilization tower **315** was connected with the tower top condenser **316** and the reflux tank **317** in sequence; an outlet of the reflux tank **317** was connected with the tail gas condenser **319,** and an outlet of the tail gas condenser **319** was connected with an inlet of the reflux tank **317;** another outlet of the reflux tank **317** was connected with the reflux pump **318,** and an outlet of the reflux pump **318** was connected with an inlet of the stabilization tower **315.**

Wherein, the first-stage reactor **311** was a fixed-bed reactor.

The apparatus for selective hydrogenation of butadiene extraction tail gas of this example was used to carry out a method for selective hydrogenation of butadiene extraction tail gas, and the selective hydrogenation method comprised:

(1) An alkyne-containing tail gas **3101** from butadiene extraction unit (based on the total weight of the alkyne-containing tail gas, the main components of the alkyne-containing tail gas were: 58.69% butene, 10.35% butadiene, 17.65% ethyl acetylene and 4.00% vinyl acetylene, 2.05% C5 and higher, 0.02% water) had a flow rate of 1825kg/h, and a pressure of 10 Kpa; before the tail gas entered the blower suction tank **31,** it was diluted with a gas phase C4 product (i.e. diluent C4 stream **3119)** produced as a side-draw from the stabilization tower, and the diluent C4 stream **3119** had a flow rate of 5000kg/h. The tail gas was fed into the blower suction tank **31,** its gas phase was pressurized to 80KPa by the blower **32,** and the C4 raw material was cooled to 5°C by the liquefaction condenser **33** using a cooling agent and then liquefied, and then fed into the C4 collection tank **34,** followed by pressurization by the booster pump **35** and being fed into the water-washing tower **36** from the bottom of the water-washing tower **36** to remove the impurities entrained in the butadiene tail gas, and then fed into the raw material tank **37** through the top of the water-washing tower **36.** The water-washing tower **36** had an operating pressure of 0.7MPaG, a washing water temperature of 40°C, and a flow rate of 8000kg/ h; the raw material tank had a pressure of 0.65 MPaG.

(2) The C4 raw material in the raw material tank **37** was pressurized to 2.7MPaG by the feed pump **38** (to obtain the C4 feed **3105),** then merged with the circulated C4 stream **3109** from the first-stage reactor outlet buffer tank **312,** and then fed into the first-stage mixer **310,** wherein it was mixed with hydrogen gas (i.e., to obtain the reactor raw material **3107),** and then fed into the first-stage reactor **311** for hydrogenation reaction, and the first-stage reaction stream obtained by the reaction (the first-stage reactor product **3108)** was fed into the first-stage reactor outlet buffer tank **312;** wherein, the circulated C4 stream **3109** had a flow rate of 45000kg/h, the mixed C4 feed had a flow rate of 51826kg/h, the first-stage reactor had an inlet temperature of 20°C, and a liquid space velocity of $20h^{-1}$.
The hydrogen gas required for the reaction in the first-stage reactor **311** was allocated and fed through a first feeding mode; the first feeding mode comprised: all the hydrogen gas required for the reaction was fed through the reactor outlet buffer tank **312,** and then fed into the first-stage reactor **311** through the first route at the outlet of the reactor outlet buffer tank **312;** wherein, the hydrogen gas required for the reaction in the first-stage reactor **311** controlled the pressure of the reaction system through pressure compensation, and the pressure was 2.3MPaG, and the hydrogen gas was fed into the liquid-phase C4 stream by the way of dissolution, and fed with the circulated C4 stream **3109** of the reactor into the first-stage reactor **311,** the circulated C4 stream **3109** had a flow rate of 45000kg/h, the hydrogen gas dissolved therein had a flow rate of 34.0kg/h, and the first-stage mixer **310** provided at the inlet of the first-stage reactor **311** was not provided with hydrogen gas.

(3) The first-stage reactor outlet buffer tank 312 had no gas-phase discharge, and the liquid phase product was divided into two streams, wherein the first stream was used as the circulated C4 stream **3109** and returned to the hydrogenation reactor **311,** and the second stream was used as the feed **3112** to the stabilization tower **315,** and had a flow rate of 6853kg/h and a temperature of 35°C.

(4) The first-stage reaction stream (the first-stage reactor product **3108)** obtained by the reaction in the first-stage reactor **311** was fed into the stabilization tower **315** through the first-stage reactor outlet buffer tank **312,** and separated by the stabilization tower **315** to produce the C4 hydrogenation product **3118;** wherein, the stabilization tower **315** was used to remove non-condensable gas **3116** from the tower top, to remove heavy components **3117** from the tower kettle, to recover the liquid-phase C4 hydrogenation product **3118** rich in 1,3-butadiene and monoolefins from the tower top, and to recover as a side-draw the gas-phase diluent C4 stream **3119** that is used for diluting raw materials and at a flow rate of 5000kg/h. The stabilization tower **15** had a theoretical plate number of 30, a side-draw position at the 25$^{th}$ theoretical plate, an operating pressure of 0.5MPaG, a tower top temperature of 56.7°C, a tower kettle temperature of 100.2°C, and a reflux (the stabilization tower reflux **3115)** flow rate of 5600kg/h.

The results of main streams were shown in Table 5.

The catalyst used in Examples 5 and 6 was: based on the total weight of the catalyst, the catalyst was composed of the following components: Pd at a content of 0.3wt%, silver metal at a content of 0.3wt%, and a carrier as the balance; in which the carrier was alumina.

Table 5

| Stream No. | 3101 | 3103 | 3108 | 3118 | 3119 | 3201 | 3202 |
|---|---|---|---|---|---|---|---|
| Phase state | Gas | Liquid | Liquid | Liquid | Gas | Gas | Gas |
| Temperature [°C] | 40 | 5 | 36.1 | 15 | 51.7 | 15.8 | 15.8 |
| Pressure [MPa(g)] | 0.01 | 0.08 | 2.3 | 0.5 | 0.01 | 2.4 | 2.4 |
| Molar composition | % | % | % | % | % | % | % |
| Hydrogen gas | 0 | 0 | 0 | 0.18 | 0 | 95 | 95 |
| Methane | 0 | 0 | 0.44 | 0.52 | 0 | 5 | 5 |
| Propane | 0 | 0 | 0 | 0 | 0 | 0 | 0 |
| Propylene | 0.01 | 0 | 0 | 0.01 | 0 | 0 | 0 |
| Propyne | 0 | 0 | 0 | 0 | 0 | 0 | 0 |
| n-Butane | 2.57 | 3 | 3.02 | 2.67 | 3.17 | 0 | 0 |
| iso-Butane | 4.67 | 1.84 | 1.83 | 4.72 | 0.8 | 0 | 0 |
| Butene-1 | 14.2 | 11.3 | 13.83 | 22.10 | 10.23 | 0 | 0 |
| iso-butene | 22.05 | 12.48 | 12.42 | 22.4 | 8.95 | 0 | 0 |
| cis-2-Butene | 18.79 | 36.16 | 36.14 | 19.35 | 42.54 | 0 | 0 |
| trans-2-Butene | 3.65 | 7.18 | 8.2 | 6.41 | 8.48 | 0 | 0 |
| 1,3-Butadiene | 6.35 | 9.76 | 12.57 | 19.37 | 11 | 0 | 0 |
| 1,2-Butadiene | 4 | 8.85 | 7.73 | 1.8 | 10.63 | 0 | 0 |
| Ethyl acetylene | 4 | 1.37 | 0.3 | 0.14 | 0.4 | 0 | 0 |
| Vinyl acetylene | 17.63 | 5.61 | 0.87 | 0.33 | 1.18 | 0 | 0 |
| n-Pentane | 1.8 | 2.39 | 2.38 | 0 | 2.61 | 0 | 0 |
| C5+ | 0.25 | 0.07 | 0.17 | 0 | 0.01 | 0 | 0 |
| Water | 0.02 | 0.01 | 0 | 0 | 0 | 0 | 0 |
| Molar flow rate [kmol/h] | 32.78 | 121.88 | 925.2 | 30.84 | 89.1 | 12.51 | 0 |
| Mass flow rate [kg/h] | 1825 | 6825 | 51826.91 | 1712.2 | 5000 | 34 | 0 |

It can be seen from Table 5 that, in the product, the content of 1,3-butadiene was about 13% higher than that in the raw material, the content of alkynes was less than 0.5%, and the total conversion rate of alkynes was greater than 97%, which could meet the raw material requirements of the butadiene extraction unit.

Example 6

As shown in Figure 3, compared with Example 5, this example had the following differences:
In step (2), the hydrogen gas required for the reaction in the first-stage reactor **311** was allocated and fed through a second feeding mode; the second feeding mode comprised: a part of the hydrogen gas required for the reaction was fed through the first-stage reactor outlet buffer tank **312,** and then fed into the first-stage reactor **311** through the first route at the outlet of the first-stage reactor outlet buffer tank **312;** the other part of the hydrogen gas was fed through the first-stage mixer **310,** and then fed into the first-stage reactor **311;** wherein, most of the hydrogen gas was allocated through the first-stage reactor outlet buffer tank **312,** and this part of the hydrogen gas controlled the pressure of the reaction system through pressure compensation, and at the same time entered the liquid-phase C4 stream through the way of dissolution, and entered the first-stage reactor along with the circulated C4 stream **3109** of the reactor, and the circulated C4 stream **3109** had a flow rate of 32000kg /h, the hydrogen gas dissolved therein had a flow rate of 20.0kg/h; a small part of the hydrogen gas is fed through the first-stage mixer **310** provided at the inlet of the first-stage reactor **311,** and had a flow rate of 15.5kg/h.

The results of main streams were shown in Table 6.

Table 6

| Stream No. | 3101 | 3103 | 3108 | 3118 | 3119 | 3201 | 3202 |
|---|---|---|---|---|---|---|---|
| Phase state | Gas | Liquid | Liquid | Liquid | Gas | Gas | Gas |
| Temperature [°C] | 40 | 5 | 41.3 | 15 | 51.5 | 15.8 | 15.8 |
| Pressure [MPa(g)] | 0.01 | 0.08 | 2.3 | 0.5 | 0.01 | 2.4 | 2.4 |
| Molar composition | % | % | % | % | % | % | % |
| Hydrogen gas | 0 | 0 | 0 | 0.18 | 0 | 95 | 95 |
| Methane | 0 | 0 | 0.48 | 0.54 | 0 | 5 | 5 |
| Propane | 0 | 0 | 0.00 | 0.00 | 0 | 0 | 0 |
| Propylene | 0.01 | 0 | 0.00 | 0.01 | 0 | 0 | 0 |
| Propyne | 0 | 0 | 0.00 | 0.00 | 0 | 0 | 0 |
| n-Butane | 2.57 | 3.02 | 3.08 | 2.69 | 3.17 | 0 | 0 |
| iso-Butane | 4.67 | 1.85 | 1.87 | 4.75 | 0.8 | 0 | 0 |
| Butene-1 | 14.2 | 11.67 | 14.32 | 23.66 | 10.23 | 0 | 0 |
| iso-butene | 22.05 | 12.52 | 12.68 | 22.58 | 8.95 | 0 | 0 |
| cis-2-Butene | 18.79 | 36.30 | 36.89 | 19.49 | 42.54 | 0 | 0 |
| trans-2-Butene | 3.65 | 7.59 | 8.48 | 6.51 | 8.48 | 0 | 0 |
| 1,3-Butadiene | 6.35 | 9.63 | 11.75 | 17.26 | 11 | 0 | 0 |
| 1,2-Butadiene | 4 | 8.30 | 7.77 | 1.80 | 10.63 | 0 | 0 |
| Ethyl acetylene | 4 | 1.31 | 0.27 | 0.13 | 0.4 | 0 | 0 |
| Vinyl acetylene | 17.63 | 5.39 | 0.75 | 0.36 | 1.18 | 0 | 0 |
| n-Pentane | 1.8 | 2.35 | 1.55 | 0.00 | 2.61 | 0 | 0 |
| C5+ | 0.25 | 0.07 | 0.13 | 0 | 0.01 | 0 | 0 |
| Water | 0.02 | 0.01 | 0 | 0 | 0 | 0 | 0 |
| Molar flow rate [kmol/h] | 32.78 | 121.84 | 693.1 | 30.81 | 89 | 7.36 | 5.70 |
| Mass flow rate [kg/h] | 1825 | 6825 | 38826.91 | 1710 | 5000 | 20 | 15.5 |

It can be seen from Table 6 that, in the product, the content of 1,3-butadiene was about 11% higher than that in the raw material, the content of alkynes was less than 0.5%, and the total conversion rate of alkynes was greater than 97%, which could meet the raw material requirements of the butadiene extraction unit. Compared with Example 5, in this example, because the hydrogen gas was not fed into the reactor completely through the way of dissolution, the even distribution of hydrogen gas was slightly worse than that of Example 5, which in turn caused a part of 1,3-butadiene to undergo hydrogenation reaction and to be consumed, resulting in a decrease in the increased value of the content of 1,3-butadiene in the product compared with the raw material.

The foregoing description has exemplarily described various embodiments of the present invention, but is not exhaustive, and the present invention is not limited to the disclosed examples. Many modifications and alterations will be apparent to those of ordinary skill in the art without departing from the scope of the described examples.

## Claims

1. A method for selective hydrogenation of butadiene extraction tail gas, **characterized in that**, the selective hydrogenation method comprises:

   (1) an alkyne-containing tail gas from a butadiene extraction unit is fed into a raw material tank, optionally impurities entrained in the alkyne-containing tail gas are removed before being fed into the raw material tank;
   (2) a C4 raw material in the raw material tank is pressurized by a feed pump to a pressure required for reaction, then merged with a circulated C4 stream from a first-stage reactor outlet buffer tank and fed into a first-stage mixer, wherein it is mixed with hydrogen gas, and fed into the first-stage reactor to undergo a first-stage hydrogenation reaction, and a first-stage reaction stream obtained by the reaction is fed into the first-stage reactor outlet buffer tank;
the hydrogen gas required for the reaction in the first-stage reactor is fed through a first feeding mode or a second feeding mode:

the first feeding mode comprises: all the hydrogen gas required for the reaction is fed through the first-stage reactor outlet buffer tank, and then fed into the first-stage reactor through a first route at an outlet of the first-stage reactor outlet buffer tank;
the second feeding mode comprises: a part of the hydrogen gas required for the reaction is fed through the first-stage reactor outlet buffer tank, and then fed into the first-stage reactor through the first route at an outlet of the first-stage reactor outlet buffer tank; and the other part of the hydrogen gas is fed through the first-stage mixer, and then fed into the first-stage reactor;

(3) there is no gas-phase discharge from the first-stage reactor outlet buffer tank, and a liquid-phase product is divided into at least two streams, wherein the first stream is returned to the first-stage reactor as the circulated C4 stream, and the second stream is used as a feed to a stabilization tower or subjected to further hydrotreatment prior to being fed into the stabilization tower;
(4) a C4 hydrogenation product is recovered after separation in the stabilization tower;

preferably, wherein, in step (2), in the second feeding mode, the mass ratio of the part of the hydrogen gas required for the reaction to the hydrogen gas required for the first-stage hydrogenation reaction is not less than 0.3, preferably not less than 0.5, and the hydrogen gas required for the first-stage hydrogenation reaction is the sum of the part of the hydrogen gas required for the reaction and the other part of the hydrogen gas.

**2.** The method for selective hydrogenation of butadiene extraction tail gas according to claim 1, wherein, in step (1), the alkyne-containing tail gas is diluted with a side-draw diluent C4 stream from the stabilization tower, preferably, the mass flow ratio of the diluent C4 stream to the alkyne-containing tail gas is 1-30:1; or
wherein, in step (1), the alkyne-containing tail gas is diluted with a diluent C4 stream from the first-stage reactor outlet buffer tank, preferably, the mass flow ratio of the diluent C4 stream to the alkyne-containing tail gas is 1-5:1.

**3.** The method for selective hydrogenation of butadiene extraction tail gas according to any one of claims 1-2, wherein, in step (3), the further hydrotreatment of the second stream prior to being fed into the stabilization tower comprises: the second stream is used as a feed to a second-stage reactor and fed into a second-stage reactor through a second-stage feed cooler and a second-stage mixer to carry out a second-stage hydrogenation reaction, and then a second-stage reaction stream obtained by the reaction in the second-stage reactor passes through a second-stage reactor outlet buffer tank and enters the stabilization tower.

**4.** The method for selective hydrogenation of butadiene extraction tail gas according to claim 3, wherein, in step (3), the hydrogen gas required for the reaction in the second-stage reactor is fed through a third feeding mode or a fourth feeding mode;

the third feeding mode comprises: all the hydrogen gas required for the reaction is fed through the first-stage reactor outlet buffer tank, and then fed into the second-stage reactor through a second route at the outlet of the first-stage reactor outlet buffer tank;
the fourth feeding mode comprises: a part of the hydrogen gas required for the reaction is fed through the first-stage reactor outlet buffer tank, and then fed into the second-stage reactor through the second route at the outlet of the first-stage reactor outlet buffer tank; and the other part of the hydrogen gas is fed through the second-stage mixer, and then fed into the second-stage reactor.
preferably, wherein, in step (3), in the fourth feeding mode, the mass ratio of the part of the hydrogen gas required for the reaction to the hydrogen gas required for the second-stage hydrogenation reaction is not less than 0.3, preferably not less than 0.5, and the hydrogen gas required for the second-stage hydrogenation reaction is the sum of the part of the hydrogen gas required for the reaction and the other part of the hydrogen gas.

**5.** The method for selective hydrogenation of butadiene extraction tail gas according to any one of claims 1-4, wherein, in step (1), the raw material tank has an operating pressure of 0.1-1.0 MPaG, preferably 0.5-1.0 MPaG; and/or

wherein, in step (2), the C4 raw material in the raw material tank is pressurized to 1.0-4.0MPaG by the feed pump, and the mass flow ratio of the circulated C4 stream to the raw material is 5-30:1; the first-stage reactor has an inlet temperature of 5-60°C, preferably 20-60°C, and a liquid space velocity of 1-50h$^{-1}$; the first-stage reactor has a pressure that is controlled by a pressure-compensating hydrogen gas of the first-stage reactor outlet buffer tank, and the reaction pressure was 1.0-4.0MPaG; and/or
wherein, in step (4), the stabilization tower has an operating pressure of 0.4-1.2 MPaG, a theoretical plate number of 10-40, and a side-draw theoretical plate position at 5-35.

**6.** The method for selective hydrogenation of butadiene extraction tail gas according to any one of claims 1-5, wherein, in step (1), the alkyne-containing tail gas from the butadiene extraction unit is fed into the raw material tank without removing the impurities from the alkyne-containing tail gas;
wherein,

in step (3), there is no gas-phase discharge from the first-stage reactor outlet buffer tank, and the liquid-phase product is divided into two streams, wherein the first stream is returned to the first-stage reactor as the circulated C4 stream, and the second stream is used as a feed to the stabilization tower;
in step (4), the first-stage reaction stream obtained from the reaction in the first-stage reactor is fed into the stabilization tower through the first-stage reactor outlet buffer tank, and separated by the stabilization tower to recover a C4 hydrogenation product.

**7.** The method for selective hydrogenation of butadiene extraction tail gas according to claim 6, wherein,

in step (1), the alkyne-containing tail gas is diluted with a side-draw diluent C4 stream from the stabilization tower, preferably, the mass flow ratio of the diluent C4 stream to the alkyne-containing tail gas is 1-30:1; and/or wherein,
in step (1), the raw material tank has an operating pressure of 0.5-1.0MPaG;
in step (2), the diluted C4 raw material is pressurized by the feed pump to 1.0-4.0MPaG, and the mass flow ratio of the circulated C4 stream to the diluted C4 raw material is 5-30:1;
the first-stage reactor has an inlet temperature of 5-60°C, and a liquid space velocity of 1-40 h$^{-1}$; the first-stage reactor has a pressure that is controlled by the pressure-compensating hydrogen gas of the first-stage reactor outlet buffer tank, and the reaction pressure is 1.0-4.0MPaG;
in step (4), the stabilization tower has an operating pressure of 0.4-1.0 MPaG, a theoretical plate number of 10-40, and a side-draw theoretical plate position at 5-35.

**8.** The method for selective hydrogenation of butadiene extraction tail gas according to any one of claims 1-5, wherein, in step (1), the alkyne-containing tail gas from the butadiene extraction unit is fed into the raw material tank without removing the impurities from the alkyne-containing tail gas;
wherein,

in step (1), the alkyne-containing tail gas is fed into the raw material tank, and the alkyne-containing tail gas in the raw material tank is diluted with the diluent C4 stream from the first-stage reactor outlet buffer tank;
in step (3), the first-stage reactor outlet buffer tank has no gas-phase discharge, and the liquid-phase product is divided into three streams, wherein the first stream is returned to the first-stage reactor as the circulated C4 stream, the second stream is used as a feed to the second-stage reactor and fed into the second-stage reactor through the second-stage feed cooler and the second-stage mixer to carry out the second-stage hydrogenation reaction, and the third stream is fed into the raw material tank as the diluent C4 stream to dilute the alkyne-containing tail gas;
the hydrogen gas required for the reaction of the second-stage reactor is fed through a third feeding mode or a fourth feeding mode:

the third feeding mode comprises: all the hydrogen gas required for the reaction is fed through the first-stage reactor outlet buffer tank, and then fed into the second-stage reactor through the second route at the outlet of the first-stage reactor outlet buffer tank;
the fourth feeding mode comprises: a part of the hydrogen gas required for the reaction is fed through the first-stage reactor outlet buffer tank, and then fed into the second-stage reactor through the second route at the outlet of the first-stage reactor outlet buffer tank; and the other part of the hydrogen gas is fed through the second-stage mixer, and then fed into the second-stage reactor;
in step (4), the second-stage reaction stream obtained by the reaction in the second-stage reactor is fed into the stabilization tower through the second-stage reactor outlet buffer tank, and separated by the stabilization tower to recover a C4 olefin product as a side-draw.

**9.** The method for selective hydrogenation of butadiene extraction tail gas according to claim 8, wherein,

in step (1), the raw material tank has an operating pressure of 0.5-1.0 MPaG, and the mass flow ratio of the diluent C4 stream to the alkyne-containing tail gas is 1-5:1;
in step (2), the diluted C4 raw material is pressurized by the feed pump to 1.0-4.0 MPaG, and the mass flow ratio of the circulated C4 stream to the diluted C4 raw material is 5-30:1;
in step (4), the stabilization tower has an operating pressure of 0.6-1.2MPaG, a theoretical plate number of 10-40, and a side-draw theoretical plate position at 5-35;
wherein, the first-stage reactor and the second-stage reactor each independently has an inlet temperature of 20-60°C, the first-stage reactor has a liquid space velocity of 10-50 $h^{-1}$, and the second-stage reactor has a liquid space velocity of 1-10 $h^{-1}$; the first-stage reactor and the second-stage reactor each independently has a pressures of 1.0-4.0MPaG that is controlled by the pressure-compensating hydrogen gas of each reactor outlet buffer tank.

**10.** The method for selective hydrogenation of butadiene extraction tail gas according to any one of claims 1-5, wherein, in step (1), the alkyne-containing tail gas is fed into a water-washing tower to remove the impurities entrained in the alkyne-containing tail gas, and then fed into the raw material tank;

preferably, wherein, in step (1), when the alkyne-containing tail gas is a gas-phase alkyne-containing tail gas, the alkyne-containing tail gas is fed into a blower suction tank, pressurized by a blower, condensed and liquefied by a liquefaction condenser, and then fed into a C4 collection tank, followed by pressurization by a booster pump, and being fed into the water-washing tower to remove the impurities entrained in the alkyne-containing tail gas, and then into the raw material tank;
preferably, wherein, in step (3), the first-stage reactor outlet buffer tank has no gas-phase discharge, and the liquid phase product is divided into two streams, wherein the first stream is returned to the first-stage reactor as the circulated C4 stream, and the second stream is used as a feed to the stabilization tower.

**11.** The method for selective hydrogenation of butadiene extraction tail gas according to claim 10, wherein,

in step (1), the alkyne-containing tail gas is diluted with a side-draw diluent C4 stream from the stabilization tower, preferably the mass flow ratio of the diluent C4 stream to the alkyne-containing tail gas is 1 to 30: 1; and/or wherein,
in step (1), the blower suction tank has an operating pressure of 0-20KPa; the condensed and liquefied gas in the liquefaction condenser has a temperature of 0-20°C, the liquefaction condenser has a pressure of 50-100KPa; the water-washing tower has an operating pressure of 0.5-1.0 MPaG, the mass ratio of the washing water in the washing tower to the C4 raw material is 1-5: 1, and the raw material tank has an operating pressure of 0.5-1.0MpaG;
in step (2), the C4 raw material is pressurized by the feed pump to 1.0-4.0MPaG; the first-stage reactor has an inlet temperature of 5-60°C, and a liquid space velocity of 10-50 $h^{-1}$; the first-stage reactor has a pressure that is controlled by the pressure-compensating hydrogen gas of the first-stage reactor outlet buffer tank, and the reaction pressure is 1.0-4.0 MPaG;
in step (4), the stabilization tower has an operating pressure of 0.4-1.0 MPaG, a theoretical plate number of 10-40, and a side-draw theoretical plate position at 5-35.

**12.** An apparatus for selective hydrogenation of butadiene extraction tail gas, which is used to carry out the method according to any one of claims 1-11, **characterized in that** the apparatus comprises: a raw material tank, a feed pump, a coalescer, a first-stage mixer, a first-stage reactor, a first-stage reactor outlet buffer tank, a circulated C4 cooler, a stabilization tower and a hydrogen gas feed pipeline;

the raw material tank, the feed pump, the coalescer, the first-stage mixer, the first-stage reactor and the first-stage reactor outlet buffer tank are connected in sequence;
an outlet pipeline of the first-stage reactor outlet buffer tank is divided into at least two routes, wherein the first route is connected with the circulated C4 cooler, the first-stage mixer and the first-stage reactor in sequence, and the second route is connected with the stabilization tower directly or indirectly;
the hydrogen gas feed pipeline is divided into at least a first pipeline and optionally a second pipeline, wherein the first pipeline is connected with the first-stage reactor outlet buffer tank, and the second pipeline is connected with the first-stage mixer;
preferably, the first-stage reactor is a fixed-bed reactor;
preferably, the first route is connected with the circulated C4 cooler though a circulation pump.

**13.** The apparatus for selective hydrogenation of butadiene extraction tail gas according to claim 12, wherein a diluent C4 port of the raw material tank is connected with an outlet of the stabilization tower, preferably an outlet of the stabilization tower is connected with the diluent C4 port through a diluent C4 pump and/or a diluent C4 cooler; or
wherein the diluent C4 port of the raw material tank is connected with an outlet pipeline of the first-stage reactor outlet buffer tank, preferably a diluent C4 cooler is further provided between the diluent C4 port of the raw material tank and the outlet pipeline of the first-stage reactor outlet buffer tank.

**14.** The apparatus for selective hydrogenation of butadiene extraction tail gas according to any one of claims 12-13, further comprising a blower suction tank, a blower, a liquefaction condenser, a C4 collection tank, a booster pump and a water-washing tower, which are connected in sequence, and a C4 raw material outlet of the water-washing tower is connected with the raw material tank.

**15.** The apparatus for selective hydrogenation of butadiene extraction tail gas according to any one of claims 12-14, further comprising a second-stage feed cooler, a second-stage mixer, a second-stage reactor and a second-stage reactor outlet buffer tank, which are connected in sequence, and the second route of the outlet pipeline of the first-stage reactor outlet buffer tank is connected with the second-stage feed cooler, and the second-stage reactor outlet buffer tank is connected with the stabilization tower;

preferably, the second route is connected with the second-stage feed cooler through a circulation pump;
preferably, the second-stage reactor is a fixed-bed reactor.

**16.** The apparatus for selective hydrogenation of butadiene extraction tail gas according to claim 15, wherein the hydrogen gas feed pipeline is divided into at least a first pipeline and optionally a second pipeline and a third pipeline, wherein the first pipeline is connected with the first-stage reactor outlet buffer tank, the second pipeline is connected with the first-stage mixer, and the third pipeline is connected with the second-stage mixer; preferably, the hydrogen gas feed pipeline further comprises a pressure-compensating pipeline that is connected with the second-stage reactor outlet buffer tank; and/or

wherein the outlet pipeline of the first-stage reactor outlet buffer tank is divided into three routes, wherein the first route is connected with the circulated C4 cooler, the first-stage mixer and the first-stage reactor in sequence, the second route is connected with the second-stage feed cooler, and the third route is connected with the diluent C4 port of the raw material tank;
the hydrogen gas feed pipeline is divided into at least a first pipeline and optionally a second pipeline and a third pipeline, wherein the first pipeline is connected with the first-stage reactor outlet buffer tank, the second pipeline is connected with the first-stage mixer, and the third pipeline is connected with the second-stage mixer.

**17.** The apparatus for selective hydrogenation of butadiene extraction tail gas according to claim 14, wherein the outlet pipeline of the first-stage reactor outlet buffer tank is divided into two routes, wherein the first route is connected with the circulated cooler, the first-stage mixer and the first-stage reactor in sequence, and the second route is connected with the stabilization tower;

preferably, wherein an outlet of the stabilization tower is connected with a diluent C4 port of the blower suction tank;
preferably, wherein the water-washing tower is provided at top with a washing water inlet and a C4 raw material outlet, and provided at bottom with a liquefied C4 raw material inlet and a washing water outlet, and the booster pump is connected with the liquefied C4 raw material inlet.

## Patentansprüche

**1.** Verfahren zur selektiven Hydrierung von Butadien-Extraktionsendgas, **dadurch gekennzeichnet, dass** das selektive Hydrierungsverfahren Folgendes umfasst:

(1) ein alkinhaltiges Endgas aus einer Butadienextraktionseinheit wird in einen Rohstofftank eingespeist, optional werden Verunreinigungen, die in dem alkinhaltigen Endgas enthalten sind, vor der Einspeisung in den Rohstofftank entfernt;

(2) ein C4-Rohstoff in dem Rohstofftank wird durch eine Einspeisepumpe auf einen für die Reaktion erforderlichen Druck gebracht, dann mit einem zirkulierten C4-Strom aus einem Erststufenreaktorauslass-Puffertank zusammengeführt und in einen ErststufenMischer eingespeist, wobei er mit Wasserstoffgas gemischt und in den Erststufenreaktor eingespeist wird, um einer Erststufen-Hydrierungsreaktion unterzogen zu werden, und ein durch die Reaktion erhaltener Erststufen-Reaktionsstrom in den Erststufenreaktorauslass-Puffertank eingespeist wird;

wobei das für die Reaktion in dem Erststufenreaktor benötigte Wasserstoffgas über einen ersten Einspeisemodus oder einen zweiten Einspeisemodus zugeführt wird:

wobei der erste Einspeisemodus Folgendes umfasst: das gesamte für die Reaktion benötigte Wasserstoffgas wird durch den Erststufenreaktorauslass-Puffertank eingespeist und dann über einen ersten Weg an einem Auslass des Erststufenreaktorauslass-Puffertanks in den Erststufenreaktor eingespeist;

wobei der zweite Einspeisemodus Folgendes umfasst: ein Teil des für die Reaktion benötigten Wasserstoffgases wird durch den Erststufenreaktorauslass-Puffertank eingespeist und wird dann über den ersten Weg an einem Auslass des Erststufenreaktorauslass-Puffertanks in den Erststufenreaktor eingespeist; und der andere Teil des Wasserstoffgases wird durch den Erststufenmischer eingespeist und dann in den Erststufenreaktor eingespeist;

(3) es gibt keinen Gasphasenabzug aus dem Erststufenreaktorauslass-Puffertank und ein Flüssigphasenprodukt wird in mindestens zwei Ströme aufgeteilt, wobei der erste Strom als der zirkulierte C4-Strom in den Erststufenreaktor zurückgeführt wird und der zweite Strom als Einspeisung in einen Stabilisierungsturm verwendet wird oder einer weiteren Hydrobehandlung unterzogen wird, bevor er in den Stabilisierungsturm eingespeist wird;

(4) nach der Abtrennung in dem Stabilisierungsturm wird ein C4-Hydrierungsprodukt zurückgewonnen;

wobei vorzugsweise in Schritt (2) in dem zweiten Eispeisemodus das Massenverhältnis des für die Reaktion erforderlichen Teils des Wasserstoffgases zu dem für die Erststufen-Hydrierungsreaktion erforderlichen Wasserstoffgas nicht weniger als 0,3 beträgt, vorzugsweise nicht weniger als 0,5, und das für die Erststufen-Hydrierungsreaktion erforderliche Wasserstoffgas die Summe des für die Reaktion erforderlichen Teils des Wasserstoffgases und des anderen Teils des Wasserstoffgases ist.

**2.** Verfahren zur selektiven Hydrierung von Butadien-Extraktionsendgas nach Anspruch 1, wobei in Schritt (1) das alkinhaltige Endgas mit einem seitlich abgezogenen C4-Verdünnungsmittelstrom aus dem Stabilisierungsturm verdünnt wird, wobei das Massenstromverhältnis des C4-Verdünnungsmittelstroms zu dem alkinhaltigen Endgas vorzugsweise 1-30:1 beträgt; oder
wobei in Schritt (1) das alkinhaltige Endgas mit einem C4-Verdünnungsmittelstrom aus dem Erststufenreaktorauslass-Puffertank verdünnt wird, wobei das Massenstromverhältnis des C4-Verdünnungsmittelstroms zu dem alkinhaltigen Endgas vorzugsweise 1-5:1 beträgt.

**3.** Verfahren zur selektiven Hydrierung von Butadien-Extraktionsendgas nach einem der Ansprüche 1-2, wobei in Schritt (3) die weitere Hydrobehandlung des zweiten Stroms vor der Einspeisung in den Stabilisierungsturm Folgendes umfasst: der zweite Strom wird als Einspeisung zu einem Zweitstufenreaktor verwendet und durch einen Zweitstufeneinspeisungskühler und einen Zweitstufenmischer in einen Zweitstufenreaktor eingespeist, um eine Zweitstufen-Hydrierungsreaktion durchzuführen, und dann fließt ein Zweitstufen-Reaktionsstrom, der durch die Reaktion in dem Zweitstufenreaktor erhalten wird, durch einen Zweitstufenreaktorauslass-Puffertank und tritt in den Stabilisierungsturm ein.

**4.** Verfahren zur selektiven Hydrierung von Butadien-Extraktionsendgas nach Anspruch 3, wobei in Schritt (3) das für die Reaktion in dem Zweitstufenreaktor benötigte Wasserstoffgas über einen dritten Einspeisemodus oder einen vierten Einspeisemodus eingespeist wird;

wobei der dritte Einspeisemodus Folgendes umfasst: das gesamte für die Reaktion benötigte Wasserstoffgas wird durch den Erststufenreaktorauslass-Puffertank eingespeist und dann über einen zweiten Weg an dem Auslass des Erststufenreaktorauslass-Puffertanks in den Zweitstufenreaktor eingespeist;

wobei der vierte Einspeisemodus Folgendes umfasst: ein Teil des für die Reaktion benötigten Wasserstoffgases wird durch den Erststufenreaktorauslass-Puffertank eingespeist und wird dann durch den zweiten Weg an dem Auslass des Erststufenreaktorauslass-Puffertanks in den Zweitstufenreaktor eingespeist; und der andere Teil des Wasserstoffgases wird durch den Zweitstufenmischer eingespeist und dann in den Zweitstufenreaktor eingespeist.

wobei vorzugsweise in Schritt (3) in dem vierten Einspeisemodus das Massenverhältnis des für die Reaktion erforderlichen Teils des Wasserstoffgases zu dem für die Zweitstufen-Hydrierungsreaktion erforderlichen Wasserstoffgas nicht weniger als 0,3 beträgt, vorzugsweise nicht weniger als 0,5, und das für die Zweitstufen-Hydrierungsreaktion erforderliche Wasserstoffgas die Summe des für die Reaktion erforderlichen Teils des Wasserstoffgases und des anderen Teils des Wasserstoffgases ist.

**5.** Verfahren zur selektiven Hydrierung von Butadien-Extraktionsendgas nach einem der Ansprüche 1-4, wobei in Schritt (1) der Rohstofftank einen Betriebsdruck von 0,1-1,0 MPaG, vorzugsweise 0,5-1,0 MPaG, aufweist; und/oder

wobei in Schritt (2) der C4-Rohstoff in dem Rohmaterialtank durch die Einspeisepumpe auf 1,0-4,0 MPaG unter Druck gesetzt wird und das Massenstromverhältnis des zirkulierten C4-Stroms zu dem Rohstoff 5-30:1 beträgt; wobei der Erststufenreaktor eine Einlasstemperatur von 5-60 °C, vorzugsweise 20-60 °C, und eine Flüssigkeitsraumgeschwindigkeit von 1-50h$^{-1}$ aufweist; wobei der Erststufenreaktor einen Druck aufweist, der durch ein druckkompensierendes Wasserstoffgas des Erststufenreaktorauslass-Puffertanks gesteuert wird, und der Reaktionsdruck 1,0-4,0 MPaG beträgt; und/oder

wobei in Schritt (4) der Stabilisierungsturm einen Betriebsdruck von 0,4-1,2 MPaG, eine theoretische Bödenanzahl von 10-40 und eine theoretische Bödenposition mit Seitenabzug an 5-35 aufweist.

**6.** Verfahren zur selektiven Hydrierung von Butadien-Extraktionsendgas nach einem der Ansprüche 1-5, wobei in Schritt (1) das alkinhaltige Endgas aus der Butadienextraktionseinheit in den Rohstofftank eingespeist wird, ohne die Verunreinigungen aus dem alkinhaltigen Endgas zu entfernen;
wobei,

es in Schritt (3) keinen Gasphasenabzug aus dem Erststufenreaktorauslass-Puffertank gibt und das Flüssigphasenprodukt in zwei Ströme aufgeteilt wird, wobei der erste Strom als der zirkulierte C4-Strom in den Erststufenreaktor zurückgeführt wird und der zweite Strom als Einspeisung zu dem Stabilisierungsturm verwendet wird;

in Schritt (4) der Erststufen-Reaktionsstrom, der aus der Reaktion in dem Erststufenreaktor erhalten wird, durch den Erststufenreaktorauslass-Puffertank in den Stabilisierungsturm eingespeist wird und durch den Stabilisierungsturm abgetrennt wird, um ein C4-Hydrierungsprodukt zurückzugewinnen.

**7.** Verfahren zur selektiven Hydrierung von Butadien-Extraktionsendgas nach Anspruch 6, wobei,

in Schritt (1) das alkinhaltige Endgas mit einem seitlich abgezogenen C4-Verdünnungsmittelstrom aus dem Stabilisierungsturm verdünnt wird, wobei das Massenstromverhältnis des C4-Verdünnungsmittelstroms zu dem alkinhaltigen Endgas vorzugsweise 1-30:1 beträgt; und/oder
wobei,
in Schritt (1) der Rohstofftank einen Betriebsdruck von 0,5-1,0 MPaG aufweist;
in Schritt (2) der verdünnte C4-Rohstoff durch die Einspeisepumpe auf 1,0-4,0 MPaG unter Druck gesetzt, und das Massenstromverhältnis des zirkulierten C4-Stroms zu dem verdünnten C4-Rohstoff 5-30:1 beträgt;
der Erststufenreaktor eine Einlasstemperatur von 5-60 °C und eine Flüssigkeitsraumgeschwindigkeit von 1-40 h$^{-1}$ aufweist; wobei der Erststufenreaktor einen Druck aufweist, der durch das druckkompensierende Wasserstoffgas des Erstsstufenreaktorauslass-Puffertanks gesteuert wird, und der Reaktionsdruck 1,0-4,0 MPaG beträgt;
in Schritt (4) der Stabilisierungsturm einen Betriebsdruck von 0,4-1,0 MPaG, eine theoretische Bödenanzahl von 10-40 und eine theoretische Bödenposition mit Seitenabzug an 5-35 aufweist.

**8.** Verfahren zur selektiven Hydrierung von Butadien-Extraktionsendgas nach einem der Ansprüche 1-5, wobei in Schritt (1) das alkinhaltige Endgas aus der Butadienextraktionseinheit in den Rohstofftank eingespeist wird, ohne die Verunreinigungen aus dem alkinhaltigen Endgas zu entfernen;
wobei,

in Schritt (1) das alkinhaltige Endgas in den Rohstofftank eingespeist wird, und das alkinhaltige Endgas in dem Rohstofftank mit dem C4-Verdünnungsmittelstrom aus dem Erststufenreaktorauslass-Puffertank verdünnt wird;

in Schritt (3) der Erststufenreaktorauslass-Puffertank keinen Gasphasenabzug aufweist und das Flüssigphasenprodukt in drei Ströme aufgeteilt wird, wobei der erste Strom als der zirkulierte C4-Strom in den Erststufenreaktor zurückgeführt wird, der zweite Strom als Einspeisung für den Zweitstufenreaktor verwendet wird und durch den Zweitstufeneinspeisungskühler und den Zweitstufenmischer in den Zweitstufenreaktor eingespeist wird, um die Zweitstufen-Hydrierungsreaktion durchzuführen, und der dritte Strom als C4-Verdünnungsmittelstrom in den Rohstofftank eingespeist wird, um das alkinhaltige Endgas zu verdünnen;
wobei das für die Reaktion des Zweitstufenreaktors benötigte Wasserstoffgas über einen dritten oder vierten Einspeisemodus eingespeist wird:

wobei der dritte Einspeisemodus Folgendes umfasst: das gesamte für die Reaktion benötigte Wasserstoffgas wird durch den Erststufenreaktorauslass-Puffertank eingespeist und dann über den zweiten Weg an dem Auslass des Erststufenreaktorauslass-Puffertanks in den Zweitstufenreaktor eingespeist;
wobei der vierte Einspeisemodus Folgendes umfasst: ein Teil des für die Reaktion benötigten Wasserstoffgases wird durch den Erststufenreaktorauslass-Puffertank eingespeist und wird dann durch den zweiten Weg an dem Auslass des Erststufenreaktorauslass-Puffertanks in den Zweitstufenreaktor eingespeist; und der andere Teil des Wasserstoffgases wird durch den Zweitstufenmischer eingespeist und dann in den Zweitstufenreaktor eingespeist;
in Schritt (4) wird der Zweitstufen-Reaktionsstrom, der durch die Reaktion in dem Zweitstufenreaktor erhalten wird, durch den Zweitstufenreaktorauslass-Puffertank in den Stabilisierungsturm eingespeist und durch den Stabilisierungsturm abgetrennt, um ein C4-Olefinprodukt als Nebenprodukt zu gewinnen.

**9.** Verfahren zur selektiven Hydrierung von Butadien-Extraktionsendgas nach Anspruch 8, wobei,

in Schritt (1) der Rohstofftank einen Betriebsdruck von 0,5-1,0 MPaG aufweist, und das Massenstromverhältnis des C4-Verdünnungsmittelstroms zu dem alkinhaltigen Endgas 1-5:1 beträgt;
in Schritt (2) der verdünnte C4-Rohstoff durch die Einspeisepumpe auf 1,0-4,0 MPaG unter Druck gesetzt wird, und das Massenstromverhältnis des zirkulierten C4-Stroms zu dem verdünnten C4-Rohstoff 5-30:1 beträgt;
in Schritt (4) der Stabilisierungsturm einen Betriebsdruck von 0,6-1,2 MPaG, eine theoretische Bödenanzahl von 10-40 und eine theoretische Bödenposition mit Seitenabzug an 5-35 aufweist;
wobei der Erststufenreaktor und der Zweitstufenreaktor jeweils unabhängig voneinander eine Einlasstemperatur von 20-60 °C aufweisen, der Erststufenreaktor eine Flüssigkeitsraumgeschwindigkeit von 10-50 $h^{-1}$ aufweist und der Zweitstufenreaktor eine Flüssigkeitsraumgeschwindigkeit von 1-10 $h^{-1}$ aufweist; der Erststufenreaktor und der Zweitstufenreaktor jeweils unabhängig voneinander einen Druck von 1,0-4,0 MPaG aufweisen, der durch das druckkompensierende Wasserstoffgas jedes Reaktorauslass-Puffertanks gesteuert wird.

**10.** Verfahren zur selektiven Hydrierung von Butadien-Extraktionsendgas nach einem der Ansprüche 1-5, wobei in Schritt (1) das alkinhaltige Endgas in einen Wasser-Waschturm eingespeist wird, um die in dem alkinhaltigen Endgas mitgeführten Verunreinigungen zu entfernen, und dann in den Rohstofftank eingespeist wird;

wobei vorzugsweise in Schritt (1), wenn das alkinhaltige Endgas ein alkinhaltiges Endgas in der Gasphase ist, das alkinhaltige Endgas in einen Gebläseabsaugtank eingespeist wird, durch ein Gebläse unter Druck gesetzt wird, durch einen Verflüssigungskondensator kondensiert und verflüssigt wird und dann in einen C4-Sammeltank eingespeist wird, anschließend durch eine Druckerhöhungspumpe unter Druck gesetzt und in den Wasser-Waschturm eingespeist wird, um die in dem alkinhaltigen Endgas enthaltenen Verunreinigungen zu entfernen, und dann in den Rohstofftank eingespeist wird;
wobei vorzugsweise in Schritt (3) der Erststufenreaktorauslass-Puffertank keinen Gasphasenabzug aufweist und das Flüssigphasenprodukt in zwei Ströme aufgeteilt wird, wobei der erste Strom als der zirkulierte C4-Strom in den Erststufenreaktor zurückgeführt wird und der zweite Strom als Zufuhr zu dem Stabilisierungsturm verwendet wird.

**11.** Verfahren zur selektiven Hydrierung von Butadien-Extraktionsendgas nach Anspruch 10, wobei,

in Schritt (1) das alkinhaltige Endgas mit einem seitlich abgezogenen C4-Verdünnungsmittelstrom aus dem Stabilisierungsturm verdünnt wird, wobei das Massenstromverhältnis des C4-Verdünnungsmittelstroms zu dem alkinhaltigen Endgas vorzugsweise 1 bis 30: 1 beträgt; und/oder
wobei,
in Schritt (1) der Gebläseabsaugtank einen Betriebsdruck von 0-20 KPa aufweist; wobei das kondensierte und verflüssigte Gas in dem Verflüssigungskondensator eine Temperatur von 0-20 °C aufweist, der Verflüssigungskondensator einen Druck von 50-100 KPa aufweist; der Wasser-Waschturm einen Betriebsdruck von 0,5-1,0 MPaG aufweist, das Massenverhältnis des Waschwassers in dem Waschturm zu dem C4-Rohstoff 1-5: 1 beträgt, und der Rohstofftank einen Betriebsdruck von 0,5-1,0 MpaG aufweist;
in Schritt (2) der C4-Rohstoff durch die Einspeisepumpe auf 1,0-4,0 MPaG unter Druck gesetzt wird; der Erststufenreaktor eine Einlasstemperatur von 5-60 °C und eine Flüssigkeitsraumgeschwindigkeit von 10-50 $h^{-1}$ aufweist; wobei der Erststufenreaktor einen Druck aufweist, der durch das druckkompensierende Wasserstoffgas des Erststufenreaktorauslass-Puffertanks gesteuert wird, und der Reaktionsdruck 1,0-4,0 MPaG beträgt;
in Schritt (4) der Stabilisierungsturm einen Betriebsdruck von 0,4-1,0 MPaG, eine theoretische Bödenanzahl von 10-40 und eine theoretische Bödenposition mit Seitenabzug an 5-35 aufweist.

**12.** Vorrichtung zur selektiven Hydrierung von Butadien-Extraktionsendgas, die zur Durchführung des Verfahrens nach einem der Ansprüche 1-11 verwendet wird, **dadurch gekennzeichnet, dass** die Vorrichtung Folgendes umfasst: einen Rohstofftank, eine Einspeisepumpe, einen Koaleszer, einen Erststufenmischer, einen Erststufenreaktor, einen Erststufenreaktorauslass-Puffertank, einen C4-Zirkulierkühler, einen Stabilisierungsturm und eine Wasserstoffgaseinspeisungsleitung;

wobei der Rohstofftank, die Einspeisepumpe, der Koaleszer, der Erststufenmischer, der Erststufenreaktor und der Erststufenreaktorauslass-Puffertank nacheinander verbunden sind;
wobei eine Auslassleitung des Erststufenreaktorauslass-Puffertanks in mindestens zwei Wege aufgeteilt ist, wobei der erste Weg nacheinander mit dem C4-Zirkulierkühler, dem Erststufenmischer und dem Erststufenreaktor verbunden ist und der zweite Weg direkt oder indirekt mit dem Stabilisierungsturm verbunden ist;
wobei die Wasserstoffgaseinspeisungsleitung in mindestens eine erste Leitung und optional eine zweite Leitung unterteilt ist, wobei die erste Leitung mit dem Erststufenreaktorauslass-Puffertank verbunden ist und die zweite Leitung mit dem Erststufenmischer verbunden ist;
wobei der Erststufenreaktor vorzugsweise ein Festbettreaktor ist;
wobei der erste Weg vorzugsweise über eine Zirkulierpumpe mit dem C4-Zirkulierkühler verbunden ist.

**13.** Vorrichtung zur selektiven Hydrierung von Butadien-Extraktionsendgas nach Anspruch 12, wobei ein C4-Verdünnungsmittelanschluss des Rohstofftanks mit einem Auslass des Stabilisierungsturms verbunden ist, wobei vorzugsweise ein Auslass des Stabilisierungsturms mit dem C4-Verdünnungsmittelanschluss durch eine C4-Verdünnungsmittelpumpe und/oder einen C4-Verdünnungsmittelkühler verbunden ist; oder
wobei der C4-Verdünnungsmittelanschluss des Rohstofftanks mit einer Auslassleitung des Erststufenreaktorauslass-Puffertanks verbunden ist, wobei weiter vorzugsweise ein C4-Verdünnungsmittelkühler zwischen dem C4-Verdünnungsmittelanschluss des Rohstofftanks und der Auslassleitung des Erststufenreaktorauslass-Puffertanks vorgesehen ist.

**14.** Vorrichtung zur selektiven Hydrierung von Butadien-Extraktionsendgas nach einem der Ansprüche 12-13, weiter umfassend einen Gebläseabsaugtank, ein Gebläse, einen Verflüssigungskondensator, einen C4-Sammeltank, eine Druckerhöhungspumpe und einen Wasser-Waschturm, die hintereinander verbunden sind, und wobei ein C4-Rohstoffauslass des Wasser-Waschturms mit dem Rohstofftank verbunden ist.

**15.** Vorrichtung zur selektiven Hydrierung von Butadien-Extraktionsendgas nach einem der Ansprüche 12-14, weiter umfassend einen Zweitstufeneinspeisekühler, einen Zweitstufenmischer, einen Zweitstufenreaktor und einen Zweitstufenreaktorauslass-Puffertank, die nacheinander verbunden sind, und wobei der zweite Weg der Auslassleitung des Erststufenreaktorauslass-Puffertanks mit dem Zweitstufeneinspeisekühler verbunden ist, und der Zweitstufenreaktorauslass-Puffertank mit dem Stabilisierungsturm verbunden ist;

wobei der zweite Weg vorzugsweise über eine Zirkulierpumpe mit dem Zweitstufeneinspeisekühler verbunden ist;
wobei der Zweitstufenreaktor vorzugsweise ein Festbettreaktor ist.

**16.** Vorrichtung zur selektiven Hydrierung von Butadien-Extraktionsendgas nach Anspruch 15, wobei die Wasserstoffgaseinspeisungsleitung in mindestens eine erste Leitung und optional eine zweite Leitung und eine dritte Leitung unterteilt ist, wobei die erste Leitung mit dem Erststufenreaktorauslass-Puffertank verbunden ist, die zweite Leitung mit dem Erststufenmischer verbunden ist und die dritte Leitung mit dem Zweitstufenmischer verbunden ist; vorzugsweise umfasst die Wasserstoffgaseinspeisungsleitung weiter eine Druckausgleichsleitung, die mit dem Zweitstufenreaktorauslass-Puffertank verbunden ist; und/oder

wobei die Auslassleitung des Erststufenreaktorauslass-Puffertanks in drei Wege unterteilt ist, wobei der erste Weg nacheinander mit dem C4-Zirkulierkühler, dem Erststufenmischer und dem Erststufenreaktor verbunden ist, der zweite Weg mit dem Zweitstufeneinspeisungskühler verbunden ist und der dritte Weg mit dem C4-Verdünnungsmittelanschluss des Rohstofftanks verbunden ist;
wobei die Wasserstoffgaseinspeisungsleitung in mindestens eine erste Leitung und optional eine zweite Leitung und eine dritte Leitung unterteilt ist, wobei die erste Leitung mit dem Erststufenreaktorauslass-Puffertank verbunden ist, die zweite Leitung mit dem Erststufenmischer verbunden ist und die dritte Leitung mit dem Zweitstufenmischer verbunden ist.

**17.** Vorrichtung zur selektiven Hydrierung von Butadien-Extraktionsendgas nach Anspruch 14, wobei die Auslassleitung des Erststufenreaktorauslass-Puffertanks in zwei Wege unterteilt ist, wobei der erste Weg nacheinander mit dem Zirkulierkühler, dem Erststufenmischer und dem Erststufenreaktor verbunden ist, und der zweite Weg mit dem Stabilisierungsturm verbunden ist;

wobei ein Auslass des Stabilisierungsturms vorzugsweise mit einem C4-Verdünnungsmittelanschluss des Gebläseabsaugtanks verbunden ist;
wobei der Wasser-Waschturm vorzugsweise an der Oberseite mit einem Waschwassereinlass und einem C4-Rohstoffauslass und an dem Boden mit einem Einlass für verflüssigten C4-Rohstoff und einem Waschwasserauslass versehen ist und die Druckerhöhungspumpe mit dem Einlass für verflüssigten C4-Rohstoff verbunden ist.

## Revendications

**1.** Procédé d'hydrogénation sélective de gaz résiduaire d'extraction de butadiène, **caractérisé en ce que**, le procédé d'hydrogénation sélective comprend :

(1) un gaz résiduaire contenant un alcyne provenant d'une unité d'extraction de butadiène est introduit dans un réservoir de matière première, les impuretés facultativement entraînées dans le gaz résiduaire contenant un alcyne étant éliminées avant qu'il ne soit introduit dans le réservoir de matière première ;
(2) une matière première C4 dans le réservoir de matière première est mise sous pression par une pompe d'alimentation à une pression requise pour la réaction, puis fusionnée avec un flux de C4 en circulation provenant d'un réservoir tampon de sortie de réacteur de premier étage et introduite dans un mélangeur de premier étage, dans lequel il est mélangé avec du gaz hydrogène, et introduit dans le réacteur de premier étage pour subir une réaction d'hydrogénation de premier étage, et un flux de réaction de premier étage obtenu par la réaction est introduit dans le réservoir tampon de sortie de réacteur de premier étage ;
le gaz hydrogène requis pour la réaction dans le réacteur de premier étage est introduit au moyen d'un premier mode d'introduction ou d'un deuxième mode d'introduction :

le premier mode d'introduction comprend : la totalité du gaz hydrogène requis pour la réaction est introduite à travers le réservoir tampon de sortie de réacteur de premier étage, et ensuite introduite dans le réacteur de premier étage à travers un premier itinéraire au niveau d'une sortie du réservoir tampon de sortie de réacteur de premier étage ;
le deuxième mode d'introduction comprend : une partie du gaz hydrogène requis pour la réaction est introduite à travers le réservoir tampon de sortie de réacteur de premier étage, et ensuite introduite dans le réacteur de premier étage à travers le premier itinéraire au niveau d'une sortie du réservoir tampon de sortie de réacteur de premier étage ; et l'autre partie du gaz hydrogène est introduite à travers le mélangeur de premier étage, et ensuite introduite dans le réacteur de premier étage ;

(3) il n'y a pas d'évacuation en phase gazeuse à partir du réservoir tampon de sortie de réacteur de premier étage, et un produit en phase liquide est divisé en au moins deux flux, dans lequel le premier flux est renvoyé vers le réacteur de premier étage en tant que le flux de C4 en circulation, et le deuxième flux est utilisé en tant qu'une alimentation d'une tour de stabilisation ou soumis à un hydrotraitement supplémentaire avant d'être introduit dans la tour de stabilisation ;
(4) un produit d'hydrogénation en C4 est récupéré après séparation dans la tour de stabilisation ;

de préférence, dans lequel, dans l'étape (2), dans le deuxième mode d'introduction, le rapport massique de la partie du gaz hydrogène requis pour la réaction au gaz hydrogène requis pour la réaction d'hydrogénation de premier étage n'est pas inférieur à 0,3, de préférence pas inférieur à 0,5, et le gaz hydrogène requis pour la réaction d'hydrogénation de premier étage est la somme de la partie du gaz hydrogène requis pour la réaction et de l'autre partie du gaz hydrogène.

**2.** Procédé d'hydrogénation sélective de gaz résiduaire d'extraction de butadiène selon la revendication 1, dans lequel, dans l'étape (1), le gaz résiduaire contenant un alcyne est dilué avec un flux de diluant C4 soutiré latéralement de la tour de stabilisation, de préférence, le rapport de débit massique du flux de diluant C4 au gaz résiduaire contenant un alcyne est 1 à 30:1 ; ou
dans lequel, dans l'étape (1), le gaz résiduaire contenant un alcyne est dilué avec un flux de diluant C4 provenant du réservoir tampon de sortie de réacteur de premier étage, de préférence, le rapport de débit massique du flux de diluant C4 au gaz résiduaire contenant un alcyne est 1 à 5:1.

**3.** Procédé d'hydrogénation sélective de gaz résiduaire d'extraction de butadiène selon l'une quelconque des revendications 1 et 2, dans lequel, dans l'étape (3), l'hydrotraitement supplémentaire du deuxième flux avant d'être introduit dans la tour de stabilisation comprend : le deuxième flux est utilisé en tant qu'une alimentation d'un réacteur de second étage et introduit dans un réacteur de second étage à travers un refroidisseur d'alimentation de second étage et un mélangeur de second étage pour réaliser une réaction d'hydrogénation de second étage, et ensuite un flux de réaction de second étage obtenu par la réaction dans le réacteur de second étage passe à travers un réservoir tampon de sortie de réacteur de second étage et entre dans la tour de stabilisation.

**4.** Procédé d'hydrogénation sélective de gaz résiduaire d'extraction de butadiène selon la revendication 3, dans lequel, dans l'étape (3), le gaz hydrogène requis pour la réaction dans le réacteur de second étage est introduit à travers un troisième mode d'introduction ou un quatrième mode d'introduction ;

le troisième mode d'introduction comprend : la totalité du gaz hydrogène requis pour la réaction est introduite à travers le réservoir tampon de sortie de réacteur de premier étage, et ensuite introduite dans le réacteur de second étage à travers un deuxième itinéraire au niveau de la sortie du réservoir tampon de sortie de réacteur de premier étage ;
le quatrième mode d'introduction comprend : une partie du gaz hydrogène requis pour la réaction est introduite à travers le réservoir tampon de sortie de réacteur de premier étage, et ensuite introduite dans le réacteur de second étage à travers le deuxième itinéraire au niveau de la sortie du réservoir tampon de sortie de réacteur de premier étage ; et l'autre partie du gaz hydrogène est introduite à travers le mélangeur de second étage, et ensuite introduite dans le réacteur de second étage ;
de préférence, dans lequel, dans l'étape (3), dans le quatrième mode d'introduction, le rapport massique de la partie du gaz hydrogène requis pour la réaction au gaz hydrogène requis pour la réaction d'hydrogénation de second étage n'est pas inférieur à 0,3, de préférence pas inférieur à 0,5, et le gaz hydrogène requis pour la réaction d'hydrogénation de second étage est la somme de la partie du gaz hydrogène requis pour la réaction et de l'autre partie du gaz hydrogène.

**5.** Procédé d'hydrogénation sélective de gaz résiduaire d'extraction de butadiène selon l'une quelconque des revendications 1 à 4, dans lequel, dans l'étape (1), le réservoir de matière première présente une pression de service de 0,1 MPaG à 1,0 MPaG, de préférence 0,5 MPaG à 1,0 MPaG ; et/ou

dans lequel, dans l'étape (2), la matière première C4 dans le réservoir de matière première est mise sous pression à 1,0 MPaG à 4,0 MPaG par la pompe d'alimentation, et le rapport de débit massique du flux de C4 en circulation à la matière première est 5 à 30:1 ; le réacteur de premier étage présente une température d'entrée de 5 °C à 60 °C, de préférence 20 °C à 60 °C, et une vitesse spatiale liquide de $1^{h-1}$ à $50^{h-1}$; le réacteur de premier étage présente une pression qui est régulée par un gaz hydrogène à compensation de pression du réservoir tampon de sortie de réacteur de premier étage, et la pression de réaction est 1,0 MPaG à 4,0 MPaG ; et/ou
dans lequel, dans l'étape (4), la tour de stabilisation présente une pression de service de 0,4 MPaG à 1,2 MPaG, un nombre de plateaux théoriques de 10 à 40, et une position de plateaux théoriques de soutirage latéral en 5 à 35.

**6.** Procédé d'hydrogénation sélective de gaz résiduaire d'extraction de butadiène selon l'une quelconque des revendications 1 à 5, dans lequel, dans l'étape (1), le gaz résiduaire contenant un alcyne provenant de l'unité d'extraction de butadiène est introduit dans le réservoir de matière première sans éliminer les impuretés du gaz résiduaire contenant un alcyne ;
dans lequel,

dans l'étape (3), il n'y a pas d'évacuation en phase gazeuse à partir du réservoir tampon de sortie de réacteur de premier étage, et le produit en phase liquide est divisé en deux flux, dans lequel le premier flux est renvoyé vers le réacteur de premier étage en tant que le flux de C4 en circulation, et le deuxième flux est utilisé en tant qu'une alimentation de la tour de stabilisation ;
dans l'étape (4), le flux de réaction de premier étage obtenu à partir de la réaction dans le réacteur de premier étage est introduit dans la tour de stabilisation à travers le réservoir tampon de sortie de réacteur de premier étage, et séparé par la tour de stabilisation pour récupérer un produit d'hydrogénation en C4.

**7.** Procédé d'hydrogénation sélective de gaz résiduaire d'extraction de butadiène selon la revendication 6, dans lequel,

dans l'étape (1), le gaz résiduaire contenant un alcyne est dilué avec un flux de diluant C4 soutiré latéralement de la tour de stabilisation, de préférence, le rapport de débit massique du flux de diluant C4 au gaz résiduaire contenant un alcyne est 1 à 30:1 ; et/ou
dans lequel,
dans l'étape (1), le réservoir de matière première présente une pression de service de 0,5 MPaG à 1,0 MPaG ;
dans l'étape (2), la matière première C4 diluée est mise sous pression par la pompe d'alimentation à 1,0 MPaG à 4,0 MPaG, et le rapport de débit massique du flux de C4 en circulation à la matière première C4 diluée est 5 à 30:1 ; le réacteur de premier étage présente une température d'entrée de 5 °C à 60 °C, et une vitesse spatiale liquide de $1^{h-1}$ à $40^{h-1}$ ; le réacteur de premier étage présente une pression qui est régulée par le gaz hydrogène à compensation de pression du réservoir tampon de sortie de réacteur de premier étage, et la pression de réaction est 1,0 MPaG à 4,0 MPaG ;
dans l'étape (4), la tour de stabilisation présente une pression de service de 0,4 MPaG à 1,0 MPaG, un nombre de plateaux théoriques de 10 à 40, et une position de plateaux théoriques de soutirage latéral en 5 à 35.

**8.** Procédé d'hydrogénation sélective de gaz résiduaire d'extraction de butadiène selon l'une quelconque des revendications 1 à 5, dans lequel, dans l'étape (1), le gaz résiduaire contenant un alcyne provenant de l'unité d'extraction de butadiène est introduit dans le réservoir de matière première sans éliminer les impuretés du gaz résiduaire contenant un alcyne ;
dans lequel,

dans l'étape (1), le gaz résiduaire contenant un alcyne est introduit dans le réservoir de matière première, et le gaz résiduaire contenant un alcyne dans le réservoir de matière première est dilué avec le flux de diluant C4 provenant du réservoir tampon de sortie de réacteur de premier étage ;
dans l'étape (3), le réservoir tampon de sortie de réacteur de premier étage ne présente pas d'évacuation en phase gazeuse, et le produit en phase liquide est divisé en trois flux, dans lequel le premier flux est renvoyé vers le réacteur de premier étage en tant que le flux de C4 en circulation, le deuxième flux est utilisé en tant qu'une alimentation du réacteur de second étage et introduit dans le réacteur de second étage à travers le refroidisseur d'alimentation de second étage et le mélangeur de second étage pour réaliser la réaction d'hydrogénation de second étage, et le troisième flux est introduit dans le réservoir de matière première en tant que le flux de diluant C4 pour diluer le gaz résiduaire contenant un alcyne ;
le gaz hydrogène requis pour la réaction du réacteur de second étage est introduit à travers un troisième mode d'introduction ou un quatrième mode d'introduction :

le troisième mode d'introduction comprend : la totalité du gaz hydrogène requis pour la réaction est introduite à travers le réservoir tampon de sortie de réacteur de premier étage, et ensuite introduite dans le réacteur de second étage à travers le deuxième itinéraire au niveau de la sortie du réservoir tampon de sortie de réacteur de premier étage ;
le quatrième mode d'introduction comprend : une partie du gaz hydrogène requis pour la réaction est introduite à travers le réservoir tampon de sortie de réacteur de premier étage, et ensuite introduite dans le réacteur de second étage à travers le deuxième itinéraire au niveau de la sortie du réservoir tampon de sortie de réacteur de premier étage ; et l'autre partie du gaz hydrogène est introduite à travers le mélangeur de second étage, et ensuite introduite dans le réacteur de second étage ;
dans l'étape (4), le flux de réaction de second étage obtenu par la réaction dans le réacteur de second étage est introduit dans la tour de stabilisation à travers le réservoir tampon de sortie de réacteur de second étage, et séparé par la tour de stabilisation pour récupérer un produit d'oléfine en C4 en tant qu'un soutirage latéral.

**9.** Procédé d'hydrogénation sélective de gaz résiduaire d'extraction de butadiène selon la revendication 8, dans lequel,

dans l'étape (1), le réservoir de matière première présente une pression de service de 0,5 MPaG à 1,0 MPaG, et le rapport de débit massique du flux de diluant C4 au gaz résiduaire contenant un alcyne est 1 à 5:1 ;
dans l'étape (2), la matière première C4 diluée est mise sous pression par la pompe d'alimentation à 1,0 MPaG à 4,0 MPaG, et le rapport de débit massique du flux de C4 en circulation à la matière première C4 diluée est 5 à 30:1 ;
dans l'étape (4), la tour de stabilisation présente une pression de service de 0,6 MPaG à 1,2 MPaG, un nombre de plateaux théoriques de 10 à 40, et une position de plateaux théoriques de soutirage latéral en 5 à 35 ;
dans lequel, le réacteur de premier étage et le réacteur de second étage présentent chacun indépendamment une température d'entrée de 20 °C à 60 °C, le réacteur de premier étage présente une vitesse spatiale liquide de $10^{h-1}$ à $50^{h-1}$, et le réacteur de second étage présente une vitesse spatiale liquide de $1^{h-1}$ à $10^{h-1}$; le réacteur de premier étage et le réacteur de second étage présentent chacun indépendamment une pression de 1,0 MPaG à 4,0 MPaG qui est régulée par le gaz hydrogène à compensation de pression de chaque réservoir tampon de sortie de réacteur.

**10.** Procédé d'hydrogénation sélective de gaz résiduaire d'extraction de butadiène selon l'une quelconque des revendications 1 à 5, dans lequel, dans l'étape (1), le gaz résiduaire contenant un alcyne est introduit dans une tour de lavage à l'eau pour éliminer les impuretés entraînées dans le gaz résiduaire contenant un alcyne, et ensuite introduit dans le réservoir de matière première ;

de préférence, dans lequel, dans l'étape (1), lorsque le gaz résiduaire contenant un alcyne est un gaz résiduaire contenant un alcyne en phase gazeuse, le gaz résiduaire contenant un alcyne est introduit dans un réservoir d'aspiration de soufflante, mis sous pression par une soufflante, condensé et liquéfié par un condenseur de liquéfaction, et ensuite introduit dans un réservoir de collecte de C4, suivi d'une mise sous pression par une pompe de surpression, et introduit dans la tour de lavage à l'eau pour éliminer les impuretés entraînées dans le gaz résiduaire contenant un alcyne, et ensuite dans le réservoir de matière première ;
de préférence, dans lequel, dans l'étape (3), le réservoir tampon de sortie de réacteur de premier étage ne présente pas d'évacuation en phase gazeuse, et le produit en phase liquide est divisé en deux flux, dans lequel le premier flux est renvoyé vers le réacteur de premier étage en tant que le flux de C4 en circulation, et le deuxième flux est utilisé en tant qu'une alimentation de la tour de stabilisation.

**11.** Procédé d'hydrogénation sélective de gaz résiduaire d'extraction de butadiène selon la revendication 10, dans lequel,

dans l'étape (1), le gaz résiduaire contenant un alcyne est dilué avec un flux de diluant C4 soutiré latéralement de la tour de stabilisation, de préférence, le rapport de débit massique du flux de diluant C4 au gaz résiduaire contenant un alcyne est 1 à 30:1 ; et/ou
dans lequel,
dans l'étape (1), le réservoir d'aspiration de soufflante présente une pression de service de 0 kPa à 20kPa ; le gaz condensé et liquéfié dans le condenseur de liquéfaction présente une température de 0 °C à 20 °C, le condenseur de liquéfaction présente une pression de 50 kPa à 100 kPa ; la tour de lavage présente une pression de service de 0,5 MPaG à 1,0 MPaG, le rapport massique de l'eau de lavage dans la tour de lavage à la matière première C4 est 1 à 5:1, et le réservoir de matière première présente une pression de service de 0,5 MPaG à 1,0 MPaG ;
dans l'étape (2), la matière première C4 est mise sous pression par la pompe d'alimentation à 1,0 MPaG à 4,0 MPaG ; le réacteur de premier étage présente une température d'entrée de 5 °C à 60 °C, et une vitesse spatiale liquide de $10^{h-1}$ à $50^{h-1}$ ; le réacteur de premier étage présente une pression qui est régulée par le gaz hydrogène à compensation de pression du réservoir tampon de sortie de réacteur de premier étage, et la pression de réaction est 1,0 MPaG à 4,0 MPaG ;
dans l'étape (4), la tour de stabilisation présente une pression de service de 0,4 MPaG à 1,0 MPaG, un nombre de plateaux théoriques de 10 à 40, et une position de plateaux théoriques de soutirage latéral en 5 à 35.

**12.** Dispositif d'hydrogénation sélective de gaz résiduaire d'extraction de butadiène, qui est utilisé pour réaliser le procédé selon l'une quelconque des revendications 1 à 11, **caractérisé en ce que** le dispositif comprend : un réservoir de matière première, une pompe d'alimentation, un coalesceur, un mélangeur de premier étage, un réacteur de premier étage, un réservoir tampon de sortie de réacteur de premier étage, un refroidisseur de C4 en circulation, une tour de stabilisation et une conduite d'introduction de gaz hydrogène ;

le réservoir de matière première, la pompe d'alimentation, le coalesceur, le mélangeur de premier étage, le réacteur de premier étage et le réservoir tampon de sortie de réacteur de premier étage sont reliés en séquence ;
une conduite de sortie du réservoir tampon de sortie de réacteur de premier étage est divisée en au moins deux itinéraires, dans lequel le premier itinéraire est relié au refroidisseur de C4 en circulation, au mélangeur de premier étage et au réacteur de premier étage en séquence, et le deuxième itinéraire est relié à la tour de stabilisation directement ou indirectement ;

la conduite d'introduction de gaz hydrogène est divisée en au moins une première conduite et facultativement une deuxième conduite, dans lequel la première conduite est reliée au réservoir tampon de sortie de réacteur de premier étage, et la deuxième conduite est reliée au mélangeur de premier étage ;

de préférence, le réacteur de premier étage est un réacteur à lit fixe ;

de préférence, le premier itinéraire est relié au refroidisseur de C4 en circulation à travers une pompe de circulation.

**13.** Dispositif d'hydrogénation sélective de gaz résiduaire d'extraction de butadiène selon la revendication 12, dans lequel un orifice de diluant C4 du réservoir de matière première est relié à une sortie de la tour de stabilisation, de préférence une sortie de la tour de stabilisation est reliée à l'orifice de diluant C4 à travers une pompe de diluant C4 et/ou un refroidisseur de diluant C4 ; ou

dans lequel l'orifice de diluant C4 du réservoir de matière première est relié à une conduite de sortie du réservoir tampon de sortie de réacteur de premier étage, de préférence un refroidisseur de diluant C4 est en outre fourni entre l'orifice de diluant C4 du réservoir de matière première et la conduite de sortie du réservoir tampon de sortie de réacteur de premier étage.

**14.** Dispositif d'hydrogénation sélective de gaz résiduaire d'extraction de butadiène selon l'une des revendications 12 et 13, comprenant en outre un réservoir d'aspiration de soufflante, une soufflante, un condenseur de liquéfaction, un réservoir de collecte de C4, une pompe de surpression et une tour de lavage à l'eau, qui sont reliés en séquence, et une sortie de matière première C4 de la tour de lavage à l'eau est reliée au réservoir de matière première.

**15.** Dispositif d'hydrogénation sélective de gaz résiduaire d'extraction de butadiène selon l'une des revendications 12 à 14, comprenant en outre un refroidisseur d'alimentation de second étage, un mélangeur de second étage, un réacteur de second étage et un réservoir tampon de sortie de réacteur de second étage, qui sont reliés en séquence, et le deuxième itinéraire de la conduite de sortie du réservoir tampon de sortie de réacteur de premier étage est relié au refroidisseur d'alimentation de second étage, et le réservoir tampon de sortie de réacteur de second étage est relié à la tour de stabilisation ;

de préférence, le deuxième itinéraire est relié au refroidisseur d'alimentation de second étage à travers une pompe de circulation ;

de préférence, le réacteur de second étage est un réacteur à lit fixe.

**16.** Dispositif d'hydrogénation sélective de gaz résiduaire d'extraction de butadiène selon la revendication 15, dans lequel la conduite d'introduction de gaz hydrogène est divisée en au moins une première conduite et facultativement une deuxième conduite et une troisième conduite, dans lequel la première conduite est reliée au réservoir tampon de sortie de réacteur de premier étage, la deuxième conduite est reliée au mélangeur de premier étage, et la troisième conduite est reliée au mélangeur de second étage ; de préférence, la conduite d'introduction de gaz hydrogène comprend en outre une conduite à compensation de pression qui est reliée au réservoir tampon de sortie de réacteur de second étage ; et/ou

dans lequel la conduite de sortie du réservoir tampon de sortie de réacteur de premier étage est divisée en trois itinéraires, dans lequel le premier itinéraire est relié au refroidisseur de C4 en circulation, au mélangeur de premier étage et au réacteur de premier étage en séquence, le deuxième itinéraire est relié au refroidisseur d'alimentation de second étage, et le troisième itinéraire est relié à l'orifice de diluant C4 du réservoir de matière première ;

la conduite d'introduction de gaz hydrogène est divisée en au moins une première conduite et facultativement une deuxième conduite et une troisième conduite, dans lequel la première conduite est reliée au réservoir tampon de sortie de réacteur de premier étage, la deuxième conduite est reliée au mélangeur de premier étage, et la troisième conduite est reliée au mélangeur de second étage.

**17.** Dispositif d'hydrogénation sélective de gaz résiduaire d'extraction de butadiène selon la revendication 14, dans lequel la conduite de sortie du réservoir tampon de sortie de réacteur de premier étage est divisée en deux itinéraires, dans lequel le premier itinéraire est relié au refroidisseur de C4 en circulation, au mélangeur de premier étage et au réacteur de premier étage en séquence, et le deuxième itinéraire est relié à la tour de stabilisation ;

de préférence, dans lequel une sortie de la tour de stabilisation est reliée à un orifice de diluant C4 du réservoir d’aspiration de soufflante ;
de préférence, dans lequel la tour de lavage à l’eau est munie au sommet d’une entrée d’eau de lavage et d’une sortie de matière première C4, et munie au bas d’une entrée de matière première C4 liquéfiée et d’une sortie d’eau de lavage, et la pompe de surpression est reliée à l’entrée de matière première C4 liquéfiée.

1116
112
114
116
1201
110
111
1118
1202
1115
1101
11
15
16
113
1107
1108
1112
13
14
12
1117
19
1109
1102
18
17
115
1112

Figure 1

2121
216
2201
218
215
214
2202
2203
2120
2101
21
25
26
212
213
217
2107
2108
2115
2116
23
24
210
211
2122
22
2109
2102
29
28
27
2123

Figure 2

3116
317
319
320
3201
315
316
3118
36
3202
311
3115
37
34
312
318
33
3107
3108
3112
39
3105
310
3101
31
3103
38
32
35
3109
3117
314
313
3119

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102285859 A
- CN 103121905 A
- CN 108863697 A
- CN 103787811 A
- CN 109806885 A
- CN 102336626 A
- CN 102285860 A
- EP 1217060 A
- CN 102240547
- CN 102886262
- CN 10886397
- CN 104707622